# EUROPEAN PATENT APPLICATION

(11) **EP 3 733 166 A1**
(43) Date of publication of application: **04.11.2020**
(21) Application number: 18896142.9
(22) Date of filing: 26.12.2018
(51) Int. Cl.: A61K 9/20, A61K 31/4178, A61K 31/4245, A61K 47/32, A61K 47/36

(54) **CYCLIC ORALLY DISINTEGRATING TABLET**

(30) Priority: 26.12.2017 JP 2017249497
(71) Applicant: TOWA PHARMACEUTICAL CO., LTD., Kadoma-shi, Osaka 5718580 (JP)
(72) Inventor: HONJO, Tatsuya, Kadoma-shi, Osaka 571-8580 (JP); SAEKI, Isamu, Kadoma-shi, Osaka 571-8580 (JP); OKUDA, Yutaka, Kadoma-shi, Osaka 571-8580 (JP)
(74) Representative: Reitstötter Kinzebach
(86) International application number: PCT/JP2018/047823
(87) International publication number: WO 2019/131753

(57) **Abstract**

Provided is an orally disintegrating tablet which can be combined with a variety of drugs and exhibits excellent disintegrating properties in an oral cavity in spite of having a strength and the like which allows a form as a tablet to be maintained. According to the present invention, provided is the annular orally disintegrating tablet containing a drug and a disintegrating agent; and having a hole in a central portion thereof and being annular, and a content of the disintegrating agent relative to a total weight thereof is 2% by weight or more and 50% by weight or less, the annular orally disintegrating tablet exhibiting the excellent disintegrating properties, in which when a gradient of a disintegrating time (seconds) of the annular orally disintegrating tablet with respect to a tableting pressure (kN) upon compression forming is defined as a and a gradient of a disintegrating time (seconds) of a disk-shaped orally disintegrating tablet with respect to a tableting pressure (kN) upon compression forming is defined as b, the disk-shaped orally disintegrating tablet having the same weight and external diameter as the weight and external diameter of the annular orally disintegrating tablet and having no hole, a ratio (a/b) between the gradient a and the gradient b is 0.90 or less.

## Description

### TECHNICAL FIELD

The present invention relates to an orally disintegrating tablet and, in particular, to an annular orally disintegrating tablet.

### BACKGROUND ART

Because in some cases, it is difficult for children and the elderly having low swallowing ability to take the conventional oral solid medicine such as a tablet and a capsule, in recent years, as a dosage form suitable for such children and the elderly, orally disintegrating tablets (OD tablets), each of which rapidly dissolves or disintegrates in an oral cavity, have been developed.

In order to rapidly disintegrate the orally disintegrating tablet in the oral cavity, it is desired in general that a tableting pressure is suppressed and a tablet hardness is lowered. However, when the tableting pressure is suppressed and the tablet hardness is lowered, there has arisen a problem in that the tablets are broken in a manufacturing process, a packaging process, or a distribution process and a yield is thereby reduced or a problem in that the tablets are broken when taken out from a packaging container and a commodity value is thereby reduced.

In addition, when the tableting pressure is increased, because rapid disintegration as the orally disintegrating tablets is impaired, achieving both of these properties which are contrary to each other has become a problem.

Therefore, as an attempt to achieve disintegrating properties in an oral cavity as an orally disintegrating tablet while a tablet hardness allowing a form as the tablet to be maintained is ensured, for example, as described in International Publication No. WO2015/053227 (Patent Literature 1), a method in which a subtle granule of a drug coated with a dissolution control film and a granulated substance constituted of an excipient, which is granulated by using crystalline cellulose, and a binder are subjected to compression forming and a tablet hardness is improved, thereby obtaining an orally disintegrating tablet whose disintegrating properties are enhanced, as described in International Publication No. WO2012/029838 (Patent Literature 2), a method in which a binder such as a methacrylic acid copolymer, which significantly increases a disintegration rate of a tablet, is added, granulation is conducted, the resultant is subjected to compression forming, and a tablet hardness is improved, thereby obtaining an orally disintegrating tablet whose disintegrating properties are enhanced, and other methods have been developed.

However, in the case of the orally disintegrating tablet described in each of Patent Literatures 1 and 2, there has arisen a problem in that it is required that the subtle granules of the drug, each coated with the dissolution control film, are prepared or that a specific binder and a disintegrating agent in an amount in accordance with an amount of the binder are blended, and kinds of additive agents to be selected, blending ratios thereof, a manufacturing process, and the like become complicated, thereby making a price of the obtained tablet extremely high. In addition, there has arisen a substantive problem in that rapid disintegration in an oral cavity is made impossible depending on kinds of the drug, and as a result, orally disintegrating tablets themselves cannot be manufactured.

### CITATION LIST

### PATENT LITERATURE

Patent Literature 1: International Publication No. WO2015/053227
Patent Literature 2: International Publication No. WO2012/029838
Patent Literature 3: Japanese Unexamined Patent Application Publication (Translation of PCT Application) No. 2009-542646

### SUMMARY OF THE INVENTION

### TECHNICAL PROBLEM

Therefore, an object of the present invention is to provide an orally disintegrating tablet which is capable of exhibiting excellent disintegrating properties in an oral cavity although the orally disintegrating tablet can be combined with a variety of drugs and has a strength which allows a form as a tablet to be maintained in a manufacturing process, a distribution process, and the like.

### SOLUTION TO PROBLEM

In order not to limit kinds of drugs to be combined as much as possible, the present inventors, et al. have devoted themselves to studies as to shortening of a disintegrating time of a tablet at the same tableting pressure at which a form of the tablet can be maintained by devising a shape of the tablet and conditions of manufacturing the tablet, instead of adding specific additive agents and the like to the tablet.

As a result, in a case of an annular tablet which is provided with a hole in the center thereof, it has been considered that because normally at the same tableting pressure, due to an increase in the tableting pressure per unit area caused by a decrease in a pressure receiving area, a density of the tablet is increased and a disintegrating time is also prolonged, as compared with a disk-shaped tablet having the same external diameter as that of the annular tablet. However, surprisingly, it was found out that in the case of the annular tablet, by subjecting the annular tablet to compression forming at a tableting pressure at which a form as a tablet can be maintained, the annular tablet exhibits more excellent disintegrating properties than those of a disk-shaped tablet which is subjected to compression forming at the same tableting pressure at which the annular tablet is subjected thereto and has the same weight and external diameter as those of the annular tablet, and the present invention was completed.

Furthermore, according to the present invention, it was found out that by making the tablet annular, even when the tableting pressure is increased, a delay of disintegration can be more suppressed than the disk-shaped tablet.

In other words, according to the present invention, provided is an annular orally disintegrating tablet which contains a drug and a disintegrating agent, has a hole in the central portion thereof, and is annular and in which a content of the disintegrating agent relative to a total weight thereof is 2% by weight or more and 50% by weight or less.

Here, when as to the annular orally disintegrating tablet, a single regression analysis regarding the relationship between a tableting pressure (kN) upon compression forming and a disintegrating time (seconds) is conducted, and a gradient of a regression formula of the disintegrating time (seconds) with respect to the obtained tableting pressure (kN) is defined as a, and as to the disk-shaped orally disintegrating tablet, which has the same weight and external diameter as those of the annular orally disintegrating tablet and has no hole, the above-mentioned similar single regression analysis is conducted, and a gradient of a regression formula of a disintegrating time with respect to the obtained tableting pressure (kN) is defined as b, it is preferable that a ratio (a/b) between the gradient a and the gradient b is 0.90 or less, and it is more preferable that the ratio therebetween is 0.50 or less.

It is to be noted that that the above-mentioned ratio (a/b) between the gradient a and the gradient b becomes smaller than 1 means that a disintegrating time of the annular orally disintegrating tablet per unit tableting pressure becomes shorter than a disintegrating time of the disk-shaped orally disintegrating tablet. Furthermore, since it is often the case that an absolute disintegrating time of the annular orally disintegrating tablet at the same tableting pressure is also shorter than the disintegrating time of the disk-shaped orally disintegrating tablet, it serves as a useful index which indicates disintegrating properties of the annular orally disintegrating tablet are excellent than those of the disk-shaped orally disintegrating tablet.

In particular, in order to make the ratio (a/b) between the gradient a of the annular orally disintegrating tablet and the gradient b of the disk-shaped orally disintegrating tablet 0.50 or less, preferably, a content of the disintegrating agent relative to a total weight of the annular orally disintegrating tablet is 5% by weight or more and 50% by weight or less, and more preferably, it is effective that the content thereof is 5% by weight or more and 30% by weight or less.

In addition, in the description of the present application, the "tableting pressure (kN)" means a tableting pressure in general upon the compression forming of tablets as long as compression forming methods for the annular orally disintegrating tablets and the disk-shaped orally disintegrating tablet, which are to be compared, are the same as each other, and the compression forming methods therefor are not limited. Therefore, the "tableting pressure (kN)" in the present invention may be, for example, a tableting pressure upon direct tableting of powder, a tableting pressure upon tableting of a granulated substance obtained after granulating the powder and for example, may be a tableting pressure upon dry compression forming or a tableting pressure upon wet compression forming.

In addition, in the description of the present application, the "disintegrating time (seconds)" means a disintegrating time in a case where as a test liquid, water is used in accordance with Disintegration Test described in the Japanese Pharmacopeia 16th Edition.

As described above, although the annular orally disintegrating tablet of the present invention is compression-formed at the tableting pressure which allows a form as the tablet to be maintained, the annular orally disintegrating tablet thereof exhibits more excellent disintegrating properties in the oral cavity than those of the disk-shaped tablet which is compression-formed at the same tableting pressure and has the same weight and external diameter. It is to be noted that in the present invention, although as a tableting pressure which is suited to maintain a form as the tablet, 4 kN or more is sufficient, in consideration of a hardness and the like of the formed tablet, it is more preferable that the tableting pressure is 4 kN to 16 kN. In addition, the annular orally disintegrating tablet of the present invention compression-formed at such a tableting pressure is capable of suppressing the disintegrating time, preferably, within 60 seconds and more preferably, within 30 seconds.

Furthermore, in order to exhibit the above-described excellent disintegrating properties, it is preferable that a ratio between an external diameter and an internal diameter of the annular orally disintegrating tablet of the present invention is 10 : 1 to 6 : 2 and it is more preferable that the ratio therebetween is 10 : 1 to 10 : 4. In addition, it is preferable that the internal diameter of the annular orally disintegrating tablet is larger than 0 mm and 4 mm or less.

In a case where the internal diameter is made constant and to be, for example, 2 mm, as long as the external diameter is within a range of 6 mm to 12 mm, the annular orally disintegrating tablet of the present invention exhibits more excellent disintegrating properties than those of the disk-shaped tablet which is compression-formed at the same tableting pressure and has the same weight and external diameter and furthermore, is capable of more suppressing a delay of disintegration than the disk-shaped tablet, even when the tableting pressure is increased.

In particular, in a case of the annular orally disintegrating tablet which has the external diameter of 10 mm and the internal diameter of 2 mm, when the disintegrating time (gradient a) thereof per unit tableting pressure is compared with the disintegrating time (gradient b) of the disk-shaped tablet, per unit tableting pressure, which is compression-formed at the same tableting pressure and has the same weight and the same external diameter, because the ratio (a/b) between the gradients becomes the smallest value, the disintegrating time of the annular orally disintegrating tablet per unit tableting pressure becomes shorter than that of the disk-shaped orally disintegrating tablet, and the annular orally disintegrating tablet exhibits excellent disintegrating properties.

In addition, in a case where the external diameter is made constant and to be, for example, 10 mm, as long as the internal diameter is within a range of 1 mm to 4 mm, the annular orally disintegrating tablet of the present invention exhibits more excellent disintegrating properties than those of the disk-shaped tablet which is compression-formed at the same tableting pressure and has the same weight and external diameter, and further surprisingly, the smaller the internal diameter is, the shorter the disintegrating time is.

By providing a score line constituted of a groove on a surface of the tablet, the annular orally disintegrating tablet of the present invention is capable of exhibiting more favorable scoring properties than those of the disk-shaped orally disintegrating tablet which is provided with a score line constituted of the same groove on a surface of the tablet. In addition, it is preferable that the score line has a straight shape or a round shape in a plan view, and it is preferable that the score line is a V-shaped groove in a cross-sectional view, an apex angle θ of the V-shaped groove being within a range of 90° ± 20°. In general, although it is often the case that one score line is provided on one surface of the tablet, the score line may be a cross-shaped score line constituted of two grooves or score lines may be provided on both surfaces.

As described above, although the annular orally disintegrating tablet of the present invention is compression-formed at the tableting pressure which allows a form as the tablet to be maintained, in a case where the annular orally disintegrating tablet of the present invention has the score line, the annular orally disintegrating tablet is capable of exhibiting more excellent scoring properties than those of the disk-shaped orally disintegrating tablet which is compression-formed under the same conditions and has the same weight and external diameter and the score line. It is to be noted that in the present invention, although as a tableting pressure which is suited to maintain a form as the tablet and allows excellent scoring properties to be exhibited, 4 kN or more is sufficient, in consideration of a hardness and the like of the formed tablet, it is preferable that the tableting pressure is 4 kN to 16 kN, and in order to obtain excellent scoring properties in particular, it is more preferable that the tableting pressure is 6 kN to 14 kN.

It is to be noted that the "scoring properties" in the description of the present application, as to 10 tablets of each of orally disintegrating tablets, scoring properties of orally disintegrating tablets were evaluated by standard deviation (%) and acceptance values (%) calculated in accordance with "2. Mass Variation Test" in "6.02 Uniformity of Dosage Units" described in the Japanese Pharmacopeia 17th Edition. The smaller the standard deviation (%) is, the smaller variation of mass of each tablet piece after scoring is, and the smaller the acceptance value (%) is, the more readily the scoring can be made, meaning that the scoring properties are excellent.

In addition, as long as the internal diameter of the annular orally disintegrating tablet, which has the score line, is constant (for example, 2 mm), regardless of external diameters (for example, within a range of 6 mm to 10 mm), as to any of the external diameters, the annular orally disintegrating tablet of the present invention exhibits more excellent scoring properties than those of the disk-shaped orally disintegrating tablet which is compression-formed at the same tableting pressure and has the same weight and the score line.

Furthermore, regardless of internal diameters (for example, within a range of 1 mm to 4 mm), at any of the tableting pressures, the annular orally disintegrating tablet of the present invention which has the score line is capable of exhibiting more excellent scoring properties than those of the disk-shaped orally disintegrating tablet which has the same weight, the same external diameter, and the score line.

In addition, regardless of tableting pressures (for example, within a range of 6 kN to 14 kN), at any of the tableting pressures, the annular orally disintegrating tablet of the present invention which has the score line is capable of exhibiting more excellent scoring properties than those of the disk-shaped orally disintegrating tablet which has the same weight and external diameter and the score line.

It is to be noted that in the description of the present application, a range of values (ratios) shown by using a word "to" indicates a range in which values before and after the word "to" are included as a minimum value and a maximum value, respectively.

As a disintegrating agent useful for enhancing the disintegrating properties of the annular orally disintegrating tablet of the present invention, polyvinyl pyrrolidone-based disintegrating agent or starch-based disintegrating agent can be cited, and both of the above-mentioned disintegrating agents may be used in combination. In addition, when the disintegrating agent is the polyvinyl pyrrolidone-based disintegrating agent, it is preferable that the polyvinyl pyrrolidone-based disintegrating agent is crospovidone. When the disintegrating agent is the starch-based disintegrating agent, it is preferable that the starch-based disintegrating agent is corn starch or sodium starch glycolate.

By making the tablet annular, instead of adding additive agents, the disintegrating properties of the annular orally disintegrating tablet of the present invention are enhanced while properties of retaining the form of the tablet, which is formed at the same tableting pressure, are maintained. Therefore, in particular, kinds of drugs which can be combined with the annular orally disintegrating tablet of the present invention are not limited, and for example, drugs such as olmesartan medoxomil and azilsartan can be favorably used.

### ADVANTAGEOUS EFFECTS OF THE INVENTION

According to the present invention, provided are an annular orally disintegrating tablet containing a drug and a disintegrating agent; and having a hole in a central portion and being annular, and a content of the disintegrating agent relative to a total weight of the annular orally disintegrating tablet is 2% by weight or more and 50% by weight or less, thereby allowing the annular orally disintegrating tablet to be combined a variety of drugs and enabling the annular orally disintegrating tablet to exhibit excellent disintegrating properties in an oral cavity in spite of having a strength which allows a form as a tablet in a manufacturing process , a distribution process, and the like to be maintained; and a method for manufacturing an annular orally disintegrating tablet.

In addition, the annular orally disintegrating tablet provided by the present invention is capable of suppressing a disintegrating time, preferably, within 60 seconds and more preferably, within 30 seconds.

Furthermore, when as to the annular orally disintegrating tablet, a single regression analysis regarding the relationship between a tableting pressure (kN) upon compression forming and a disintegrating time (seconds) is conducted, and a gradient of a regression formula of the disintegrating time (seconds) with respect to the obtained tableting pressure (kN) is defined as a, and as to a disk-shaped orally disintegrating tablet, which has the same weight and external diameter as those of the annular orally disintegrating tablet and has no hole, the above-mentioned similar single regression analysis is conducted, and a gradient of a regression formula of a disintegrating time with respect to the obtained tableting pressure (kN) is defined as b, it is preferable that a ratio (a/b) between the gradient a and the gradient b is 0.90 or less, and it is more preferable that the ratio therebetween is 0.50 or less. Therefore, a delay of disintegration caused by an increase in the tableting pressure is suppressed, and as a result, both of prevention of breakage and chipping of tablets and excellent disintegrating properties can be achieved by adopting a high tableting pressure.

By providing a score line constituted of a groove on a surface of the tablet, the annular orally disintegrating tablet of the present invention is capable of exhibiting more favorable scoring properties than those of the disk-shaped orally disintegrating tablet which is provided with a score line constituted of the similar groove on a surface of the tablet.

### BRIEF DESCRIPTION OF THE DRAWINGS

FIG. 1 shows a front view and a side view of an annular orally disintegrating tablet in Example 14 which has an external diameter of 10 mm, an internal diameter of 1 mm, and a tablet weight of 360 mg.
FIG. 2 shows a front view and a side view of an annular orally disintegrating tablet in Example 4 which has an external diameter of 10 mm, an internal diameter of 2 mm, and a tablet weight of 360 mg.
FIG. 3 shows a front view and a side view of an annular orally disintegrating tablet in Example 15 which has an external diameter of 10 mm, an internal diameter of 3 mm, and a tablet weight of 360 mg.
FIG. 4 shows a front view and a side view of an annular orally disintegrating tablet in Example 16 which has an external diameter of 10 mm, an internal diameter of 4 mm, and a tablet weight of 360 mg.
FIG. 5 shows a front view and a side view of a disk-shaped orally disintegrating tablet in Comparative Example 4 which has an external diameter of 10 mm and a tablet weight of 360 mg.
FIG. 6 is a graph showing the relationship between a disintegrating time and a tableting pressure of each of disintegrating tablets in Example 5 and Comparative Example 5, in which as a drug, olmesartan medoxomil is blended and an added amount of crospovidone is 30% by weight.
FIG. 7 is a graph showing the relationship between a disintegrating time and a tableting pressure of each of disintegrating tablets in Example 9 and Comparative Example 9, in which as a drug, azilsartan is blended and an added amount of crospovidone is 30% by weight.
FIG. 8 is a graph showing the relationship between a disintegrating time and a tableting pressure of each of disintegrating tablets in Test Example 10 and Comparative Example 10, in which no drug is blended and crospovidone of 30% by weight is added.
FIG. 9 is a graph showing the relationship between a disintegrating time and a tableting pressure of each of an annular orally disintegrating tablet in Test Example 0 and a disk-shaped orally disintegrating tablet in Comparative Example 0, in which a blended amount of crospovidone is 0% by weight.
FIG. 10 is a graph showing the relationship between a disintegrating time and a tableting pressure of each of an annular orally disintegrating tablets in Example 1 and a disk-shaped orally disintegrating tablet in Comparative Example 1, in which an added amount of crospovidone is 2% by weight.
FIG. 11 is a graph showing the relationship between a disintegrating time and a tableting pressure of each of an annular orally disintegrating tablet in Example 2 and a disk-shaped orally disintegrating tablet in Comparative Example 2, in which a blended amount of crospovidone is 3% by weight.
FIG. 12 is a graph showing the relationship between a disintegrating time and a tableting pressure of each of an annular orally disintegrating tablet in Example 3 and a disk-shaped orally disintegrating tablet in Comparative Example 3, in which an added amount of crospovidone is 5% by weight.
FIG. 13 is a graph showing the relationship between a disintegrating time and a tableting pressure of each of an annular orally disintegrating tablet in Example 4 which has an external diameter of 10 mm and an internal diameter of 2 mm and a disk-shaped orally disintegrating tablet in Comparative Example 4 which has an external diameter of 10 mm, in which a blended amount of crospovidone is 10% by weight.
FIG. 14 is a graph showing the relationship between a disintegrating time and a tableting pressure of each of an annular orally disintegrating tablet in Example 6 and a disk-shaped orally disintegrating tablet in Comparative Example 6, in which an added amount of the crospovidone is 50% by weight.
FIG. 15 is a graph showing the relationship between a disintegrating time and a tableting pressure of each of an annular orally disintegrating tablet in Example 7 and a disk-shaped orally disintegrating tablet in Comparative Example 7, in which as a disintegrating agent, corn starch of 50% by weight is blended.
FIG. 16 is a graph showing the relationship between a disintegrating time and a tableting pressure of each of an annular orally disintegrating tablet in Example 8 and a disk-shaped orally disintegrating tablet in Comparative Example 8, in which as a disintegrating agent, Primojel of 50% by weight is blended.
FIG. 17 shows a front view and a side view of an annular orally disintegrating tablet in Example 11 which has an external diameter of 6 mm, an internal diameter of 2 mm, and a tablet weight of 360 mg.
FIG. 18 shows a front view and a side view of an annular orally disintegrating tablet in Example 12 which has an external diameter of 8 mm, an internal diameter of 2 mm, and a tablet weight of 360 mg.
FIG. 19 shows a front view and a side view of an annular orally disintegrating tablet in Example 13 which has an external diameter of 12 mm, an internal diameter of 2 mm, and a tablet weight of 360 mg.
FIG. 20 is a graph showing the relationship between a disintegrating time and a tableting pressure of each of the annular orally disintegrating tablet in Example 11 which has the external diameter of 6 mm and the internal diameter of 2 mm and a disk-shaped orally disintegrating tablet in Comparative Example 11 which has an external diameter of 6 mm.
FIG. 21 is a graph showing the relationship between a disintegrating time and a tableting pressure of each of the annular orally disintegrating tablet in Example 12 which has the external diameter of 8 mm and the internal diameter of 2 mm and a disk-shaped orally disintegrating tablet in Comparative Example 12 which has an external diameter of 8 mm.
FIG. 22 is a graph showing the relationship between a disintegrating time and a tableting pressure of each of the annular orally disintegrating tablet in Example 13 which has the external diameter of 12 mm and the internal diameter of 2 mm and a disk-shaped orally disintegrating tablet in Comparative Example 13 which has an external diameter of 12 mm.
FIG. 23 is a graph showing the relationship between a disintegrating time and a tableting pressure of each of orally disintegrating tablets whose each external diameter is 10 mm and whose internal diameters of holes are 0 mm (without a hole), 1 mm, 2 mm, 3 mm, and 4 mm, respectively.
FIG. 24 shows a front view and a side view of an annular orally disintegrating tablet in Example 17R (having a round score line) which has an external diameter of 6 mm, an internal diameter of 2 mm, and a tablet weight of 90 mg.
FIG. 25 shows a front view and a side view of an annular orally disintegrating tablet in Example 18R (having a round score line) which has an external diameter of 8 mm, an internal diameter of 2 mm, and a tablet weight of 180 mg.
FIG. 26 shows a front view and a side view of each of annular orally disintegrating tablets in Examples 19R and 22R to 26R (having a round score line), which has an external diameter of 10 mm, an internal diameter of 2 mm, and a tablet weight of 360 mg.
FIG. 27 shows a front view and a side view of each of annular orally disintegrating tablets in Examples 19S and 22S (having a straight score line), which has an external diameter of 10 mm, an internal diameter of 2 mm, and a tablet weight of 360 mg.
FIG. 28 shows a front view and a side view of each of disk-shaped orally disintegrating tablets in Comparative Examples 16R and 20R to 24R (having a round score line), which has an external diameter of 10 mm and a tablet weight of 360 mg.
FIG. 29 shows a front view and a side view of a disk-shaped orally disintegrating tablet in Comparative Example 14S (having a straight score line), which has an external diameter of 6 mm and a tablet weight of 90 mg.
FIG. 30 shows a front view and a side view of a disk-shaped orally disintegrating tablet in Comparative Example 15S (having a straight score line), which has an external diameter of 8 mm and a tablet weight of 180 mg.
FIG. 31 shows a front view and a side view of each of disk-shaped orally disintegrating tablets in Comparative Examples 16S and 20S (having a straight score line), which has an external diameter of 10 mm and a tablet weight of 360 mg.
FIG. 32 shows a front view and a side view of an annular orally disintegrating tablet in Example 20R (having a round score line), which has an external diameter of 10 mm, an internal diameter of 1 mm, and a tablet weight of 360 mg.
FIG. 33 shows a front view and a side view of an annular orally disintegrating tablet in Example 21R (having a round score line), which has an external diameter of 10 mm, an internal diameter of 4 mm, and a tablet weight of 360 mg.

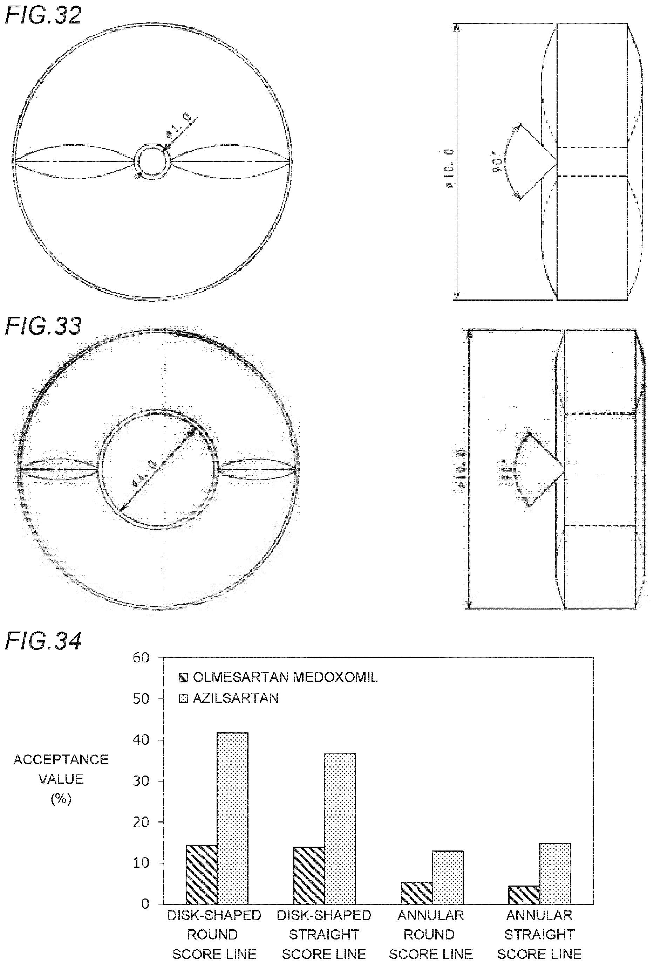

FIG. 34 is a graph showing comparison of scoring properties, made for annular orally disintegrating tablets in Examples 19R and 22R (each having a round score line), whose each external diameter is 10 mm and whose each internal diameter is 2 mm and disk-shaped orally disintegrating tablets in Comparative Examples 16R and 20R (each having a round score line), whose each external diameter is 10 mm, and annular orally disintegrating tablets in Examples 19S and 22S (each having a straight score line), whose each external diameter of 10 mm and disk-shaped orally disintegrating tablets in Comparative Examples 16S and 20S (each having a straight score line).
FIG. 35 is a graph showing comparison of scoring properties, made for annular orally disintegrating tablets in Examples 17R, 18R, and 19R (each having a round score line), whose external diameters are 6 mm, 8 mm, and 10 mm, respectively and whose each internal diameter is 2 mm, and disk-shaped orally disintegrating tablets in Comparative Examples 14S, 15S, and 16S (each having a straight score line), whose external diameters are 6 mm, 8 mm, and 10 mm, respectively.
FIG. 36 is a graph showing comparison of scoring properties, made for annular orally disintegrating tablets in Examples 20R, 19R, and 21R (each having a round score line), whose each external diameter is 10 mm and whose internal diameters of holes are 1 mm, 2 mm, and 4 mm, respectively and a disk-shaped orally disintegrating tablet in Comparative Example 16S (having a straight score line) whose external diameter is 10 mm and whose internal diameter of a hole is 0 (without a hole).
FIG. 37 is a graph showing the relationship between scoring properties and a tableting pressure of each of annular orally disintegrating tablets in Examples 22R to 26R (having a round score line), whose external diameter is 10 mm and whose internal diameter is 2 mm and each of disk-shaped orally disintegrating tablets in Comparative Examples 20R to 24R (having a round score line), whose external diameter is 10 mm.

### DESCRIPTION OF EMBODIMENTS

Hereinafter, an annular orally disintegrating tablet and a method for manufacturing an annular orally disintegrating tablet according to one embodiment of the present invention will be described in detail with reference to the accompanying drawings. It is to be noted that the present invention is not limited to Examples described below, and various modifications can be made within the scope of the technical idea of the present invention.

The annular orally disintegrating tablet of the present invention may include a drug, a disintegrating agent, and additive agents such as an excipient, a binder, a fluidizer, a sweetening agent, and a lubricant.

Although the drug in the present invention is not limited as long as the drug can be orally administered as the drug in the annular orally disintegrating tablet, for example, used is or are a component or components of one kind or two or more kinds selected from the group consisting of an antipyretic analgesic antiphlogistic drug; a psychotropic drug; an anti-anxiety drug; an antidepressant drug; a hypnotic sedative drug; an antispasmodic drug; a central nervous system acting drug; a brain metabolic stimulant; a cerebral circulation activator; an antiepileptic drug; a sympathomimetic drug; a gastrointestinal drug; an acid suppressant drug; an antipeptic ulcer agent; an antitussive expectorant agent; an antiemetic; a respiratory stimulant; a bronchodilator; allergy medicine; an agent for dental and oral use; an antihistaminic drug; a cardiotonic agent; an antiarrhythmic agent; a diuretic; an antihypertensive agent; a vasoconstrictor agent; a coronary vessel dilator; a peripheral vessel dilator; an agent for hyperlipidemia; a cholagogue; an antibiotic drug; a chemotherapeutic agent; an antidiabetic agent; a drug for osteoporosis; an antirheumatic drug; an antispasmodic agent; a hormone drug; an alkaloidal narcotic; a sulfa drug; a gout remedy; an anticoagulant; an antineoplastic agent; a nutritional fortification health drug; and the like.

More specifically, olmesartan, azilsartan, amlodipine, and like and pharmacologically acceptable salts thereof or prodrugs thereof and the like can be used as the drug which can be blended in the annular orally disintegrating tablet of the present invention. In particular, since olmesartan medoxomil is a prodrug which is rapidly hydrolyzed when absorbed, whose olmesartan as an active metabolite is separated therefrom, and which exhibits drug efficacy, the term simply referred to as olmesartan medoxomil in the description of the present application means that "olmesartan" is included.

When the drug in the present invention is any of the pharmacologically acceptable salts, as the pharmacologically acceptable salts, for example, a pharmacologically acceptable amino acid addition salt, metal salt, ammonium salt, organic amine addition salt, amino acid addition salt, and the like are included. As the pharmacologically acceptable amino acid addition salt, for example, cited are an inorganic acid salt such as hydrochloride, hydrobromide, a nitrate salt, hydrosulfate, and phosphate, and for example, an organic acid salt such as acetate, maleate, fumarate, tartrate, and citrate. As the pharmacologically acceptable metal salt, for example, cited are an alkali metal salt such as a lithium salt, a sodium salt, and a potassium salt, and for example, an alkali earth metal salt such as a magnesium salt and a calcium salt, and an aluminum salt, and a zinc salt. As the pharmacologically acceptable ammonium salt, for example, cited are salts of ammonium, tetramethylammonium, and the like. As the pharmacologically acceptable organic amine addition salt, for example, cited are addition salts of morpholine, piperidine, and the like. As the pharmacologically acceptable amino acid addition salt, for example, cited are addition salts of lysine, glycine, phenylalanine, asparagine acid, glutamic acid, and the like.

As the disintegrating agent which can be added to the annular orally disintegrating tablet of the present invention, for example, cited are starch (for example, corn starch, potato starch, rice starch, wheat starch, pregelatinized starch, partially pregelatinized starch, and the like), starch derivative (carboxymethyl starch sodium, hydroxypropyl starch, and the like), low-substituted hydroxypropylcellulose, carmellose, carmellose calcium, croscarmellose sodium, crospovidone, bentonite, and the like, and each of these may be used singly or two or more kinds of these may be used. It is to be noted that each of the above-mentioned disintegrating agents may serve for other uses such as an excipient and/or a binder.

More specifically, for example, sugar (for example, white sugar, maltose, and the like), sugar alcohol (for example, sorbitol and the like), cellulose (for example, crystalline cellulose, powdered cellulose, and the like), hardly water-soluble inorganic salt (for example, talc, an light anhydrous silicic acid, and the like), and the like can be used as the disintegrating agent which can be added to the annular orally disintegrating tablet of the present invention, and each of these may be used singly or two or more kinds of these may be used.

As the binder which can be added to the annular orally disintegrating tablet of the present invention, for example, cited are cellulose derivative (for example, methyl cellulose, carmellose, carboxypropyl cellulose, hydroxypropyl cellulose, hydroxypropyl methyl cellulose, and the like), cellulose (for example, crystalline cellulose and the like), starch (for example, pregelatinized starch and the like), polyvinyl alcohol, polyvinyl pyrrolidone, pullulan, dextrin, gum arabic, gelatin, and the like, and each of these may be used singly or two or more kinds of these may be used.

As the fluidizer which can be added to the annular orally disintegrating tablet of the present invention, for example, cited are hydrous silicon dioxide, light anhydrous silicic acid, synthetic aluminum silicate, heavy anhydrous silicic acid, alumina magnesium hydroxide, stearic acid, calcium stearate, magnesium stearate, talc, magnesium aluminometasilicate, and the like, and each of these may be used singly or two or more kinds of these may be used.

As the sweetening agent which can be added to the annular orally disintegrating tablet of the present invention, for example, cited are aspartame, erythritol, fruit sugar, xylitol, brown sugar, saccharin. saccharin sodium hydrate, sucralose, refined white sugar, refined white sugar spherical granules, D-sorbitol, dextrates, white sugar, glucose, maltitol, maltose hydrate, D-mannitol, thaumatin, and the like, and each of these may be used singly or two or more kinds of these may be used.

As the lubricant which can be added to the annular orally disintegrating tablet of the present invention, for example, cited are magnesium stearate, calcium stearate, sodium stearyl fumarate, hardened oil, sucrose fatty acid ester, polyethylene glycol, and the like, and more preferably, cited are magnesium stearate, calcium stearate, sodium stearyl fumarate, and the like, and each of these may be used singly or two or more kinds of these may be used.

In addition, the annular orally disintegrating tablet of the present invention may contain a coloring agent, a light blocking agent, and a flavoring agent, and more specifically, the annular orally disintegrating tablet can contain, for example, a titanium oxide, an iron oxide (specifically, a yellow ferric oxide, an iron sesquioxide, a yellow iron oxide, a black iron oxide, and the like), a zinc oxide, a silicon oxide, a red iron oxide, a carbon black, medicinal carbon, barium sulfate, edible yellow No. 4 aluminum lake, food red No. 2, food red No. 3, food red No. 102, copper chlorophyllin, a variety of flavoring agents, and the like.

As a shape of the annular orally disintegrating tablet in the present invention, the annular orally disintegrating tablet may be a round tablet, a triangular tablet, a cannonball-shaped tablet, or the like as long as the annular orally disintegrating tablet has a hole in a central portion thereof, and a size of the tablet is also not particularly limited. However, in order to enhance disintegrating properties in the oral cavity while a strength required to maintain a form as the tablet is ensured, it is preferable that a ratio of an external diameter and an internal diameter of the annular orally disintegrating tablet is 10 : 1 to 6 : 2 and it is more preferable that the ratio thereof is 10 : 1 to 10 : 4. In addition, it is preferable that the internal diameter of the annular orally disintegrating tablet is more than 0 mm and 4 mm or less.

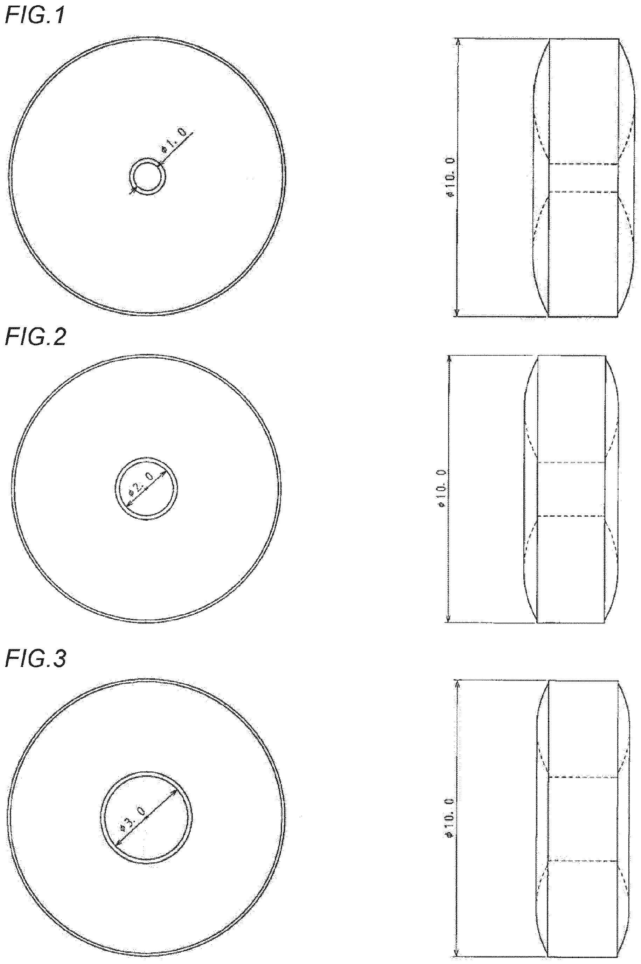

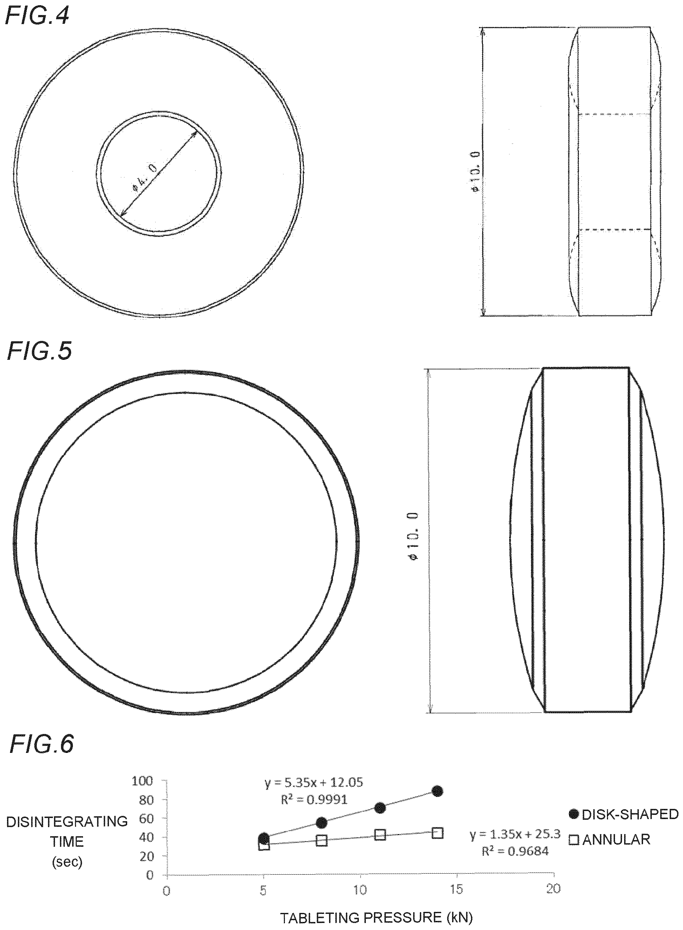

Specifically, as the annular orally disintegrating tablet having the hole in the central portion thereof, it is preferable that the annular orally disintegrating tablet has an external diameter of 10 mm, an internal diameter of 4 mm, and a tablet weight of 360 mg, shown in FIG. 4; it is more preferable that the annular orally disintegrating tablet has an external diameter of 10 mm, an internal diameter of 3 mm, and a tablet weight of 360 mg, shown in FIG. 3; it is further preferable that the annular orally disintegrating tablet has an external diameter of 10 mm, an internal diameter of 2 mm, and a tablet weight of 360 mg, shown in FIG. 2; and it is the most preferable that the annular orally disintegrating tablet has an external diameter of 10 mm, an internal diameter of 1 mm , and a tablet weight of 360 mg, shown in FIG. 1.

In addition, in a case where the internal diameter of the annular orally disintegrating tablet of the present invention is made constant and to be, for example, 2 mm, when the external diameter is within a range of 6 mm to 12 mm, the above-mentioned annular orally disintegrating tablet exhibits more excellent disintegrating properties than those of a disk-shaped tablet which is compression-formed at the same tableting pressure and has the same weight and external diameter as those of the annular orally disintegrating tablet, and even when the tableting pressure is further increased, the annular orally disintegrating tablet is capable of more suppressing a delay of disintegration than the disk-shaped tablet.

It is to be noted that in the present embodiment, a disk-shaped orally disintegrating tablet in Comparative Example which has no hole in a central portion thereof, has an external diameter of 10 mm and a tablet weight of 360 mg as shown in FIG. 5 was prepared and by comparing the above-mentioned disk-shaped orally disintegrating tablet with the annular orally disintegrating tablets in Examples of the present invention shown in FIGS. 1 to 4, evaluation as to effect of the annular orally disintegrating tablets of the present invention at respective tableting pressures in enhancement of disintegrating properties was conducted.

A method for manufacturing the annular orally disintegrating tablet of the present invention is a method for manufacturing the annular orally disintegrating tablet which contains the drug, the disintegrating agent, and the excipient, and for example, cited is a method for manufacturing the annular orally disintegrating tablet, which includes a process of tableting a mixed powder in which these additive agents are mixed and a granulated substance obtained by dry granulation, in which slugging by roller compression or the like and pulverization are conducted, or a granulated substance obtained by adding a binder liquid to the excipient and conducting wet granulation, by using a pestle having a protrusion for making a hole.

For the dry granulation, for example, cited are a roller compression method (by a dry granulation apparatus or the like), a slugging method (a rotary type tableting machine or the like), a dry particle composing method (a dry particle composing machine or the like) in which particle composing, surface modification, and spheronization are conducted under dry conditions, and the like.

As a method of adding the binder liquid to the excipient and conducting the wet granulation, for example, cited are an extrusion granulation method (by a screw extrusion granulation apparatus, a roll extrusion type granulation apparatus, or the like), a tumbling granulation method (by a rotary drum type granulation apparatus, a centrifugal tumbling type granulation apparatus, or the like), a fluidized bed granulation method (by a fluidized bed granulation drying apparatus, a tumbling fluidized bed granulation apparatus, or the like), an agitation granulation method (by an agitation granulation apparatus or the like), and the like. Preferably, cited are the fluidized bed granulation method, the agitation granulation method, and the like. More preferably, the fluidized bed granulation method is cited. In any of the above-mentioned cases, a method in which the binder liquid is added thereto and the granulation is conducted and the granulated substance is dried is preferable. In addition, as a method of adding the binder liquid thereto, a spraying addition method is preferable. In the present invention, the fluidized bed granulation method is a method in which while hot air is sent from a lower part of the granulation apparatus and a powder and granular material is fluidized, the binder liquid is sprayed, thereby conducting the granulation. In addition, for example, a spray facing type method in which the binder liquid is sprayed from above and a parallel type (Wurster type) method in which the binder liquid is sprayed from below are included, and a tumbling type fluidized bed granulation method in which a lower part of a container of a granulation apparatus rotates is also included.

In addition, when the binder liquid is added to the excipient and the wet granulation is conducted, wet granulation in which the drug and/or the disintegrating agent, other active components and/or other additive agents as desired are mixed together with the excipient and the binder liquid is added thereto may be conducted.

The annular orally disintegrating tablet of the present invention is manufactured by tableting the obtained mixed powder and granulated substance by means of the tableting apparatus by using the pestle having the protrusion for making the hole in the central portion of the tablet. When the drug and/or the disintegrating agent are not blended in the granulated substance, the drug and/or the disintegrating agent are mixed with the obtained granulated substance in another process, and the resultant is tableted by means of the above-mentioned tableting apparatus, also thereby allowing the annular orally disintegrating tablet to be manufactured. In any of the cases, other additive agents such as the excipient and/or the binder are further mixed as desired, and tableting is conducted by the above-mentioned tableting apparatus, also thereby allowing the annular orally disintegrating tablet to be manufactured.

It is only required for the tableting apparatus, which can be used in the present invention, to use the pestle having the protrusion for making the hole in the central portion of the tablet, and for example, a tableting apparatus such as a rotary tableting apparatus and a hydraulic press apparatus can be used. In addition, for example, the so-called external lubrication tableting method in which a tableting apparatus having a pestle and a mortar to which an infinitesimal amount of a lubricant constituted of stearic acid such as stearic acid, magnesium stearate, and calcium stearate or metal salts thereof, sucrose fatty acid ester, glycerin fatty acid ester, hardened oil, or the like is applied is employed, also thereby allowing the annular orally disintegrating tablet to be manufactured.

It is only required for a tableting pressure in the tableting process in the present invention to be a pressure capable of imparting a strength which allows a form as the tablet in the manufacturing process, the distribution process, and the like to be maintained, and for example, the tableting is conducted at a pressure of 5 kN to 20 kN and preferably, at a pressure of 5 kN to 15 kN.

Next, the present invention will be specifically described with reference to Examples and Test Examples. However, the present invention is not limited to these Examples and Test Examples.

### 1. Effect of making a shape of an orally disintegrating tablet annular (with a hole)

### (1) Manufacturing of orally disintegrating tablets

Annular orally disintegrating tablets in Examples 5 and 9, in each of which as a drug, olmesartan medoxomil or azilsartan was used; an annular orally disintegrating tablet in Test Example 10 in which no drug was blended; disk-shaped orally disintegrating tablets in Comparative Examples 5 and 9, in each of which as a drug, the olmesartan medoxomil or the azilsartan was used; and a disk-shaped orally disintegrating tablet in Comparative Example 10 in which no drug is blended were manufactured as described below. Amounts of respective components are as shown in Tables 1 to 3. In addition, as a main form of the annular orally disintegrating tablet in each of Examples 5 and 9 and Test Example 10, an external diameter was 10 mm, an internal diameter was 2 mm, and a tablet weight was 360 mg (see FIG. 2). In addition, as a main form of the disk-shaped orally disintegrating tablet in each of Comparative Examples 5, 9, and 10, an external diameter was 10 mm and a tablet weight was 360 mg (see FIG. 5).

The annular orally disintegrating tablet in Example 5 in which as the drug, the olmesartan medoxomil was used was manufactured by inputting the olmesartan medoxomil and a lactose hydrate into a fluidized bed granulation apparatus, spraying an aqueous solution of hydroxypropyl cellulose thereto, conducting granulation, drying, and particle size regulation, and thereafter, adding mannitol and crospovidone thereto and mixing the resultant, and further adding magnesium stearate thereto and mixing the resultant, and tableting the resultant by means of a hydraulic press apparatus by using a pestle having an external diameter of 10 mm and an internal diameter of 2 mm. In addition, the disk-shaped orally disintegrating tablet in Comparative Example 5 was manufactured under the same manufacturing conditions as those under which the annular orally disintegrating tablet in Example 5 was manufactured except that a pestle having no protrusion for making a hole in a central portion of the tablet and having an external diameter of 10 mm.

Components of the annular orally disintegrating tablet in Example 5 in which as the drug, the olmesartan medoxomil was used and components of the disk-shaped orally disintegrating tablet in Comparative Example 5 are shown in Table 1.

**[Table 1]**

| (Blended amount: mg) | | | |
|---|---|---|---|
| | | Example 5 | Comparative Example 5 |
| Shape (external diameter/internal diameter: mm) | | Annular/with a hole (10/2) | Disk-shaped/without a hole (10/-) |
| Intra-granular part | Olmesartan medoxomil | 40 | |
| | Lactose hydrate | 40 | |
| | Hydroxypropyl cellulose | 1 | |
| Intra-granular part total | | 81 | |
| Extra-granular part | Mannitol | 167.4 | |
| | Crospovidone (30 wt%) | 108 | |
| | Magnesium stearate | 3.6 | |
| Total (tablet weight) | | 360 | |

The annular orally disintegrating tablet in Example 9 in which as the drug, the azilsartan was used was manufactured by inputting the azilsartan, mannitol, hydroxypropyl cellulose, low-substituted hydroxypropylcellulose, and polyethyleneglycol into an agitation granulation apparatus, spraying an aqueous dispersion of a yellow iron sesquioxide thereto, conducting granulation, and conducting drying and particle size regulation by means of a fluidized bed granulation apparatus, and thereafter, adding mannitol and crospovidone and mixing the resultant, and further adding magnesium stearate thereto and mixing the resultant, and tableting the resultant by means of a hydraulic press apparatus by using a pestle having an external diameter of 10 mm and an internal diameter of 2 mm. In addition, the disk-shaped orally disintegrating tablet in Comparative Example 9 was manufactured under the same manufacturing conditions as those under which the annular orally disintegrating tablet in Example 9 was manufactured except that a pestle having no protrusion for making a hole in a central portion of the tablet and having an external diameter of 10 mm.

Components of the annular orally disintegrating tablet in Example 9 in which as the drug, the azilsartan was used and components of the disk-shaped orally disintegrating tablet in Comparative Example 9 are shown in Table 2.

**[Table 2]**

| (Blended amount: mg) | | | |
|---|---|---|---|
| | | Example 9 | Comparative Example 9 |
| Shape (external diameter/internal diameter: mm) | | Annular/with a hole (10/2) | Disk-shaped/without a hole (10/-) |
| Intra-granular part | Azilsartan | 20 | |
| | Mannitol | 8.9 | |
| | Hydroxypropyl cellulose | 6.6 | |
| | Low substituted hydroxypropylcellulose | 2.3 | |
| | Polyethyleneglycol | 2 | |
| | Yellow iron sesquioxide | 0.2 | |
| Intra-granular part total | | 40 | |
| Extra-granular part | Mannitol | 208.4 | |
| | Crospovidone (30 wt%) | 108 | |
| | Magnesium stearate | 3.6 | |
| Total (tablet weight) | | 360 | |

The annular orally disintegrating tablet in Test Example 10 in which no drug was blended was manufactured by adding mannitol and crospovidone and mixing the resultant, further adding magnesium stearate thereto and mixing the resultant, and tableting by means of a hydraulic press apparatus by using a pestle having an external diameter of 10 mm and an internal diameter of 2 mm. In addition, the disk-shaped orally disintegrating tablet in Comparative Example 10 was manufactured under the same manufacturing conditions as those under which the annular orally disintegrating tablet in Test Example 10 was manufactured except that a pestle having no protrusion for making a hole in a central portion of the tablet and having an external diameter of 10 mm.

Components of the annular orally disintegrating tablet in Test Example 10 in which no drug was blended and components of the disk-shaped orally disintegrating tablet in Comparative Example 10 are shown in Table 3.

**[Table 3]**

| (Blended amount: mg) | | | |
|---|---|---|---|
| | | Test Example 10 | Comparative Example 10 |
| Shape (external diameter/internal diameter: mm) | | Annular/with a hole (10/2) | Disk-shaped/without a hole (10/-) |
| Extra-granular part | Mannitol | 248.4 | |
| | Crospovidone (30 wt%) | 108 | |
| | Magnesium stearate | 3.6 | |
| Total (tablet weight) | | 360 | |

### (2) Disintegrating properties of orally disintegrating tablets

Disintegrating properties of the orally disintegrating tablets were evaluated by measuring disintegrating times (seconds) of the tablets in a case where as a test liquid, water was used in accordance with a Disintegration Test method described in the Japanese Pharmacopeia 16th Edition.

In addition, as to each of the annular orally disintegrating tablets, a single regression analysis regarding the relationship between a tableting pressure (kN) upon compression forming and the above-mentioned disintegrating time (seconds) was conducted, and a gradient of a regression formula of the disintegrating time (seconds) with respect to the obtained tableting pressure (kN) was defined as a. As to each of the disk-shaped orally disintegrating tablets, which had the same weight and external diameter as those of each of the annular orally disintegrating tablets and had no hole, the above-mentioned similar single regression analysis was conducted, and a gradient of a regression formula of a disintegrating time (seconds) with respect to the obtained tableting pressure (kN) was defined as b. By calculating a ratio (a/b) of the gradient a to the gradient b, effect in suppression of a delay of disintegration due to an increase in the tableting pressure of each of the annular orally disintegrating tablets was evaluated. In other words, although when the tableting pressure is increased, it is thereby made possible to maintain a strength of the tablet and to reduce breakage and the like, on the other hand, the disintegrating time is prolonged (a delay of disintegration). Here, that the ratio (a/b) of the gradient a to the gradient b becomes smaller than 1 indicates that even when the tableting pressure is increased, the delay of disintegration is hardly caused by the annular orally disintegrating tablet, as compared with the disk-shaped orally disintegrating tablet. Furthermore, since it is often the case that an absolute disintegrating time of the annular orally disintegrating tablet formed at the same tableting pressure is also shorter than that of the disk-shaped orally disintegrating tablet, thereby, as a whole, resulting in more excellent disintegrating properties of the annular orally disintegrating tablet than those of the disk-shaped orally disintegrating tablet.

Hereinafter, as to the disintegrating properties of each of the annular orally disintegrating tablets and each of the disk-shaped orally disintegrating tablets, one evaluation was conducted by comparing the disintegrating times in accordance with the Disintegration Test method of the Japanese Pharmacopeia 16th Edition. As to each of the annular orally disintegrating tablets and each of the disk-shaped orally disintegrating tablets, the other evaluation was conducted by obtaining a ratio (a/b) of the disintegrating time per unit tableting pressure obtained from the relationship between the tableting pressure and the disintegrating time.

Evaluation results of the disintegrating properties of the annular orally disintegrating tablets in Examples 5 and 9 and Test Example 10 and the disintegrating properties of the disk-shaped orally disintegrating tablets in Comparative Examples 5, 9, and 10 are shown in Table 4 and FIGS. 6 to 8.

**[Table 4]**

| | | Example 5 | Comparative Example 5 | Example 9 | Comparative Example 9 | Test Example 10 | Comparative Example 10 |
|---|---|---|---|---|---|---|---|
| Drug | | Olmesartan medoxomil | | Azilsartan | | - | |
| Shape | | Annular | Disk-shaped | Annular | Disk-shaped | Annular | Disk-shaped |
| Disintegrating time (seconds) | Tableting pressure 5 kN | 32 | 39 | 18 | 20 | 17 | 18 |
| | Tableting pressure 8 kN | 36 | 55 | 21 | 27 | 21 | 25 |
| | Tableting pressure 11 kN | 42 | 70 | 24 | 42 | 28 | 36 |
| | Tableting pressure 14 kN | 44 | 88 | 25 | 55 | 31 | 47 |
| Regression formula (correlation coefficient R²) | | y=1.35x+25.3 R²=0.9684 | y=5.35x+12.05 R²=0.9991 | y=0.8x+14.4 R²=0.96 | y=4x-2 R²=0.9809 | y=1.6333x+8.7333 R²=0.978 | y=3.2667x+0.4667 R²=0.9901 |
| Gradient | | a=1.35 | b=5.35 | a=0.8 | b=4 | a=1.6333 | b=3.2667 |
| a/b | | 0.25 | | 0.20 | | 0.50 | |

It was found out from Table 4 and FIGS. 6 to 8 that when Example 5 and Comparative Example 5, Example 9 and Comparative Example 9, and Test Example 10 and Comparative Example 10, which are basically different from each other only in the shapes of the tablets which are annular and disk-shaped, respectively are compared, the disintegrating time of each of the annular orally disintegrating tablets in Examples 5 and 9 and Test Example 10 becomes shorter than that of each of the disk-shaped orally disintegrating tablets in Comparative Examples 5 and 9 and 10 at the same tableting pressure, respectively. In other words, although regardless of kinds of the drugs and presence/absence of the blended drug, each of the annular orally disintegrating tablets has a strength, which allows a form as the same tablet as that of each of the disk-shaped orally disintegrating tablets formed at the same tableting pressure, to be maintained, each of the annular orally disintegrating tablets exhibits more excellent disintegrating properties than those of each of the disk-shaped orally disintegrating tablets. It is to be noted that although as a tableting pressure which allows excellent disintegrating properties to be exhibited while a form as the tablet is maintained, 5 kN or more is sufficient, in consideration of a hardness and the like of each of the actually formed tablets, it is more preferable that the tableting pressure is 5 kN to 14 kN.

In addition, as to the relationship between the above-mentioned tableting pressure and the disintegrating time, when the gradient a of the regression formula of each of the annular orally disintegrating tablets obtained by the single regression analysis and the gradient b of the regression formula of each of the disk-shaped orally disintegrating tablets were compared (a/b), it was found out that because the comparison (a/b) of the gradient a and the gradient b of each of the annular orally disintegrating tablets in Examples 5 and 9 and Test Example 10 was smaller than 1.0, the disintegrating time per unit tableting pressure of each of the annular orally disintegrating tablets was short and each of the annular orally disintegrating tablets has more excellent disintegrating properties than those of each of the disk-shaped orally disintegrating tablets (FIGS. 6 to 8).

### 2. Influence exerted by blending of disintegrating agent

### (1) Manufacturing of orally disintegrating tablets

In order to study influence exerted on disintegrating times of each of the annular orally disintegrating tablets and each of the disk-shaped orally disintegrating tablets formed at the same tableting pressure by presence and absence of addition of a disintegrating agent and a blended amount thereof, the below-described test was conducted. Specifically, annular orally disintegrating tablets in Test Example 0 and Examples 1 to 4 and 6, each of which had an external diameter of 10 mm, an internal diameter of 2 mm, and a tablet weight of 360 mg (see FIG. 2) and disk-shaped orally disintegrating tablets in Comparative Examples 0 to 4 and 6, each of which had an external diameter of 10 mm and a tablet weight of 360 mg were manufactured by using olmesartan medoxomil as the drug and employing the manufacturing method in which the annular orally disintegrating tablet in Example 5 or the disk-shaped orally disintegrating tablet in Comparative Example 5 was manufactured under the same manufacturing conditions as those under which the annular orally disintegrating tablet in Example 5 or the disk-shaped orally disintegrating tablet in Comparative Example 5 was manufactured, except that blended amounts of crospovidone as the disintegrating agent were 0% by weight, 2% by weight, 3% by weight, 5% by weight, 10% by weight, 30% by weight, 50% by weight in respective tablet weights.

Components of the annular orally disintegrating tablets in Test Example 0 and Examples 1 to 6 and the disk-shaped orally disintegrating tablet in Comparative Examples 0 to 6, whose blended amounts of the crospovidone were 0% by weight, 2% by weight, 3% by weight, 5% by weight, 10% by weight, 30% by weight, 50% by weight in the respective tablet weights are shown in Table 5.

**[Table 5]**

| (Blended amount: mg) | | | | | | | | |
|---|---|---|---|---|---|---|---|---|
| | | Test Example 0/Comparative Example 0 | Example 1/Comparative Example 1 | Example 2/Comparative Example 2 | Example 3/Comparative Example 3 | Example 4/Comparative Example 4 | Example 5/Comparative Example 5 | Example 6/Comparative Example 6 |
| Shape | | (Annular/with a hole)/(Disk-shaped/without a hole) | | | | | | |
| Intra-granular part | Olmesartan medoxomil | 40 | 40 | 40 | 40 | 40 | 40 | 40 |
| | Lactose hydrate | 40 | 40 | 40 | 40 | 40 | 40 | 40 |
| | Hydroxypropyl cellulose | 1 | 1 | 1 | 1 | 1 | 1 | 1 |
| Intra-granular part total | | 81 | 81 | 81 | 81 | 81 | 81 | 81 |
| Extra-granular part | Mannitol | 275.4 | 268.2 | 264.6 | 257.4 | 239.4 | 167.4 | 95.4 |
| | Crospovidone | 0 (0 wt%) | 7.2 (2 wt%) | 10.8 (3 wt%) | 18 (5 wt%) | 36 (10 wt%) | 108 (30 wt%) | 180 (50 wt%) |
| | Magnesium stearate | 3.6 | 3.6 | 3.6 | 3.6 | 3.6 | 3.6 | 3.6 |
| Total (tablet weight) | | 360 | 360 | 360 | 360 | 360 | 360 | 360 |

### (2) Disintegrating properties of orally disintegrating tablets

As to disintegrating properties of the annular orally disintegrating tablets in Test Example 0 and Examples 1 to 6 and the disk-shaped orally disintegrating tablets in Comparative Examples 0 to 6, as described in the above-mentioned "1. Effect of making a shape of an orally disintegrating tablet annular (with a hole)", one evaluation was conducted by comparing disintegrating times in accordance with the Disintegration Test method of the Japanese Pharmacopeia 16th Edition. As to each of the annular orally disintegrating tablets and each of the disk-shaped orally disintegrating tablets, the other evaluation was conducted by obtaining a ratio (a/b) of the disintegrating time per unit tableting pressure obtained from the relationship between the tableting pressure and the disintegrating time.

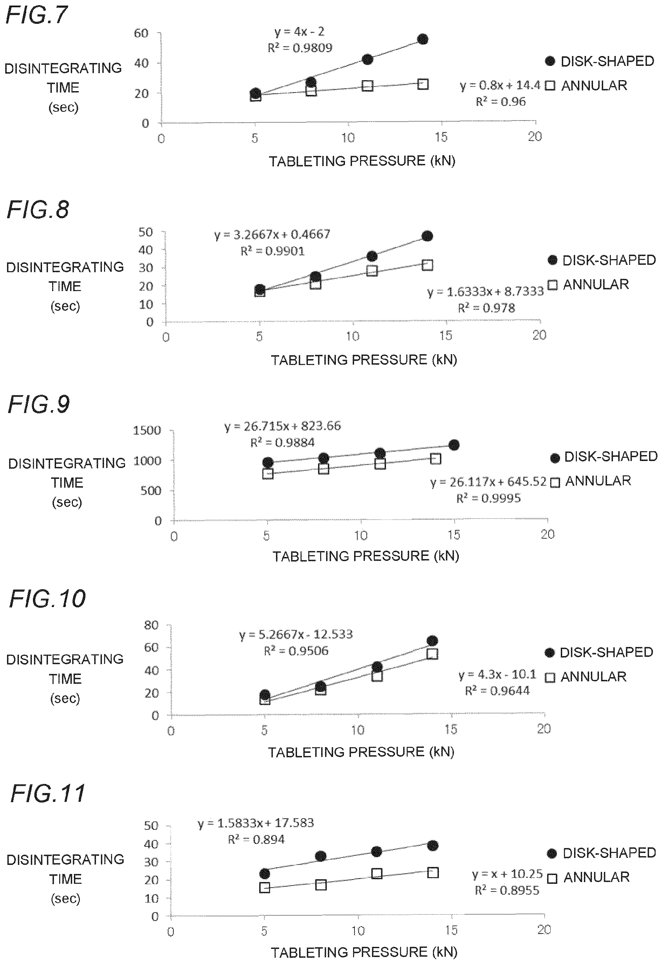

Evaluation results of disintegrating properties as to the annular orally disintegrating tablets in Test Example 0 and Examples 1 to 6 and the disk-shaped orally disintegrating tablets in Comparative Examples 0 to 6 are shown in Table 6 and FIGS. 9 to 13, 6, and 14.

**[Table 6]**

| | | Test Example 0 | Example 1 | Example 2 | Example 3 | Example 4 | Example 5 | Example 6 |
|---|---|---|---|---|---|---|---|---|
| Disintegrating agent blended amount | | 0 wt% | 2 wt% | 3 wt% | 5 wt% | 10 wt% | 30 wt% | 50 wt% |
| Shape (external diameter/internal diameter: mm) | | Annular /with a hole (10/2) | | | | | | |
| Disintegrating time (seconds) | Tableting pressure 5 kN | 778 | 14 | 16 | 16 | 19 | 32 | 63 |
| | Tableting pressure 8 kN | 853 | 22 | 17 | 17 | 23 | 36 | 90 |
| | Tableting pressure 11 kN | 931 | 34 | 23 | 22 | 26 | 42 | 117 |
| | Tableting pressure 14 kN | 1013 | 53 | 24 | 27 | 32 | 44 | 147 |
| Regression formula (correlation coefficient R²) | | y=26.117x+645.52 R²=0.9995 | y=4.3x-10.1 R²=0.9644 | y=x+10.25 R²=0.8955 | y=1.2333x+8.5333 R²=0.9219 | y=1.45x+11.1 R²=0.9836 | y=1.35x+25.3 R²=0.9684 | y=9.2333x+16.283 R²=0.9992 |
| Gradient | | a=26.117 | a=4.3 | a=1 | a=1.2333 | a=1.45 | a=1.35 | a=9.2333 |
| | | Comparative Example 0 | Comparative Example 1 | Comparative Example 2 | Comparative Example 3 | Comparative Example 4 | Comparative Example 5 | Comparative Example 6 |
| Disintegrating agent blended amount | | 0 wt% | 2 wt% | 3 wt% | 5 wt% | 10 wt% | 30 wt% | 50 wt% |
| Shape (external diameter/internal diameter: mm) | | Disk-shaped /without a hole (10/-) | | | | | | |
| Disintegrating time (seconds) | Tableting pressure 5 kN | 966 | 18 | 24 | 32 | 27 | 39 | 64 |
| | Tableting pressure 8 kN | 1034 | 25 | 33 | 49 | 40 | 55 | 112 |
| | Tableting pressure 11 kN | 1102 | 42 | 36 | 61 | 70 | 70 | 151 |
| | Tableting pressure 14 kN | 1235 | 65 | 39 | 78 | 101 | 88 | 177 |
| Regression formula (correlation coefficient R²) | | y=26.715x+823.66 R²=0.9884 | y=5.2667x-12.533 R²=0.9506 | y=1.5833x+17.583 R²=0.894 | y=5x+7.5 R²=0.9956 | y=8.4167x-20.583 R²=0.9693 | y=5.35x+12.05 R²=0.9991 | y=12.617x+5.7667 R²=0.9824 |
| Gradient | | b=26.715 | b=5.2667 | b=1.5833 | b=5 | b=8.4167 | b=5.35 | b=12.617 |
| a/b | | 0.98 | 0.82 | 0.63 | 0.25 | 0.17 | 0.25 | 0.73 |

It was found out from test results of the annular orally disintegrating tablet in Test Example 0 and the disk-shaped orally disintegrating tablet in Comparative Example 0 shown in Table 6 and FIG. 9 that when the blended amount of the crospovidone as the disintegrating agent was made to be 0% by weight in the tablet weight of each thereof, the disintegrating times of the annular orally disintegrating tablet and the disk-shaped orally disintegrating tablet formed at the same respective tableting pressures hardly make differences. In addition, it was found out that also as to the relationship between the tableting pressure and the disintegrating time, because when the comparison (a/b) between the gradient a of a regression formula of the annular orally disintegrating tablet in Test Example 0 and the gradient b of a regression formula of the disk-shaped orally disintegrating tablet in Comparative Example 0, obtained by the single regression analysis, was made, the a/b was 0.98, also as to the disintegrating times per unit tableting pressure of the annular orally disintegrating tablet and the disk-shaped orally disintegrating tablet, differences were hardly made. In other words, it was found out that when the blended amount of the crospovidone as the disintegrating agent was made to be 0% by weight in the tablet weight of each thereof, as compared with the disk-shaped orally disintegrating tablets formed at the same tableting pressure, shortening effect of the disintegrating time was hardly obtained by making the shape of the tablet annular with the hole.

On the other hand, it was found out from test results of the annular orally disintegrating tablets in Examples 1 to 6 and the disk-shaped orally disintegrating tablets in Comparative Examples 1 to 6, shown in Table 6 and FIGS. 10 to 13, 6, and 14 that when the blended amounts of the crospovidone as the disintegrating agent were respectively made to be 2% by weight or more and 50% by weight or less in the tablet weight, in most cases, disintegrating times of the annular orally disintegrating tablets formed at the same respective tableting pressures became shorter than disintegrating times of the disk-shaped orally disintegrating tablets formed at the same respective tableting pressures. In addition, it was found out that as to the relationship between the tableting pressure and the disintegrating time, because when the comparison (a/b) between a gradient of a regression formula of each of the annular orally disintegrating tablets in Examples 1 to 6 and the gradient b of a regression formula of each of the disk-shaped orally disintegrating tablets in Comparative Examples 1 to 6, obtained by the single regression analysis, was made, the a/b became 0.90 or less, the disintegrating time of each of the annular orally disintegrating tablets per unit tableting pressure became shorter than the disintegrating time of each of the disk-shaped orally disintegrating tablets, and each of the annular orally disintegrating tablets had excellent disintegrating properties. It is to be noted that although as a tableting pressure which allows excellent disintegrating properties to be exhibited while a form as the tablet is maintained, 5 kN or more is sufficient, in consideration of a hardness and the like of each of the actually formed tablets, it is more preferable that the tableting pressure is 5 kN to 14 kN.

In addition, it was found out from test results of the annular orally disintegrating tablets in Examples 2 to 5 in particular that because when the blended amounts of the crospovidone as the disintegrating agent were made to be 5% by weight or more and 30% by weight or less in the tablet weight, as to the relationship between the tableting pressure and the disintegrating time, a ratio (a/b) of a gradient of each of the annular orally disintegrating tablets and each of the disk-shaped orally disintegrating tablets became 0.50 or less, as to the disintegrating time, each of the annular orally disintegrating tablets per unit tableting pressure had extremely excellent disintegrating properties.

In addition, it was found out that by making the blended amount of the crospovidone to be 2% by weight or more and 30% by weight% or less in the tablet weight of each of the annular orally disintegrating tablets of the present invention, it was made possible to suppress the disintegrating time within 60 seconds (Examples 1 to 5), and more preferably, by making the blended amount of the crospovidone to be 3% by weight or more and 10% by weight or less in the tablet weight, it was made possible to suppress the disintegrating time substantially within 30 seconds (Examples 2 to 4).

### 3. Influence exerted by kinds of disintegrating agents

### (1) Manufacturing of orally disintegrating tablets

In order to study influence exerted on disintegrating times of annular orally disintegrating tablets and disk-shaped orally disintegrating tablets by kinds of blended disintegrating agents, formed at the same tableting pressure, the below-described test was conducted. Specifically, annular orally disintegrating tablets in Examples 7 and 8, each of which had an external diameter of 10 mm, an internal diameter of 2 mm, and a tablet weight of 360 mg (see FIG. 2), and disk-shaped orally disintegrating tablets in Comparative Examples 7 and 8, each of which had an external diameter of 10 mm and a tablet weight of 360 mg, were manufactured by employing the same manufacturing method in which the annular orally disintegrating tablet in Example 6 or the disk-shaped orally disintegrating tablet in Comparative Example 6 was manufactured and under the same manufacturing conditions as those under which the annular orally disintegrating tablet in Example 6 or the disk-shaped orally disintegrating tablet in Comparative Example 6 was manufactured, except that as the disintegrating agent, corn starch or Primojel was used.

Components of the annular orally disintegrating tablets in Examples 6 to 8 and components of the disk-shaped orally disintegrating tablets in Comparative Examples 6 to 8, in each of which as the disintegrating agent, the crospovidone, the corn starch, or Primojel was used, are shown in Table 7.

**[Table 7]**

| (Blended amount: mg) | | | | |
|---|---|---|---|---|
| | | Example 6/Comparative Example 6 | Example 7/Comparative Example 7 | Example 8/Comparative Example 8 |
| Shape | | (Annular/with a hole)/(Disk-shaped/without a hole) | | |
| Intra-granular part | Olmesartan medoxomil | 40 | 40 | 40 |
| | Lactose hydrate | 40 | 40 | 40 |
| | Hydroxypropyl cellulose | 1 | 1 | 1 |
| Intra-granular part total | | 81 | 81 | 81 |
| Extra-granular part | Mannitol | 275.4 | 268.2 | 264.6 |
| | Disintegrating agent (50 wt%) | Crospovidone 180 | Corn starch 180 | Primojel 180 |
| | Magnesium stearate | 3.6 | 3.6 | 3.6 |
| Total (tablet weight) | | 360 | 360 | 360 |

### (2) Disintegrating properties of orally disintegrating tablets

As to disintegrating properties of the annular orally disintegrating tablets in Examples 6 to 8 and the disk-shaped orally disintegrating tablets in Comparative Examples 6 to 8, as described in the above-mentioned "1. Effect of making a shape of an orally disintegrating tablet annular (with a hole)", one evaluation was conducted by comparing disintegrating times in accordance with the Disintegration Test method of the Japanese Pharmacopeia 16th Edition. As to each of the annular orally disintegrating tablets and each of the disk-shaped orally disintegrating tablets, the other evaluation was conducted by obtaining a ratio (a/b) of the disintegrating time per unit tableting pressure obtained from the relationship between the tableting pressure and the disintegrating time.

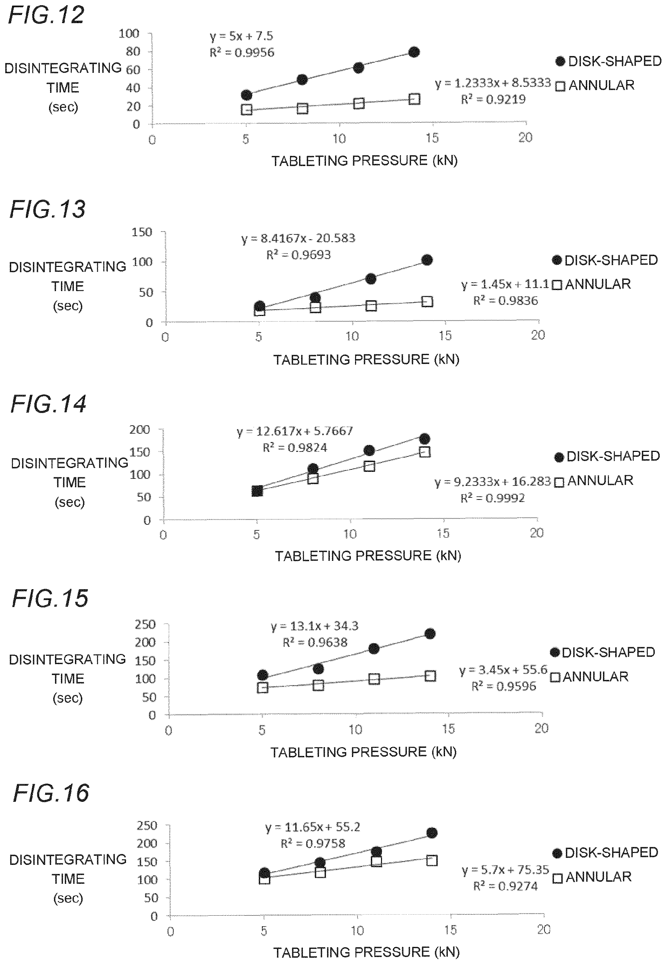

Evaluation results of the disintegrating properties as to the annular orally disintegrating tablets in Examples 6 to 8 and the disk-shaped orally disintegrating tablets in Comparative Examples 6 to 8 are shown in Table 8 and FIGS. 14 to 16.

**[Table 8]**

| | | Example 6 | Example 7 | Example 8 |
|---|---|---|---|---|
| Disintegrating agent (50 wt%) | | Crospovidone | Corn starch | Primojel |
| Shape (external diameter/internal diameter: mm) | | Annular /with a hole (10/2) | | |
| Disintegrating time (seconds) | Tableting pressure 5 kN | 63 | 75 | 103 |
| | Tableting pressure 8 kN | 90 | 80 | 119 |
| | Tableting pressure 11 kN | 117 | 96 | 147 |
| | Tableting pressure 14 kN | 147 | 104 | 150 |
| Regression formula (correlation coefficient R²) | | y=9.2333x+16.283 | y=3.45x+55.6 | y=5.7x+75.35 |
| | | R²=0.9992 | R²=0.9596 | R²=0.9274 |
| Gradient | | a=9.2333 | a=3.45 | a=5.7 |
| | | Comparative Example 6 | Comparative Example 7 | Comparative Example 8 |
| Disintegrating agent (50 wt%) | | Crospovidone | Corn starch | Primojel |
| Shape (external diameter/internal diameter: mm) | | Disk-shaped /without a hole (10/-) | | |
| disintegrating time (seconds) | Tableting pressure 5 kN | 64 | 109 | 119 |
| | Tableting pressure 8 kN | 112 | 125 | 145 |
| | Tableting pressure 11 kN | 151 | 181 | 175 |
| | Tableting pressure 14 kN | 177 | 221 | 225 |
| Regression formula (correlation coefficient R²) | | y=12.617x+5.7667 | y=13.1x+34.3 | y=11.65x+55.2 |
| | | R²=0.9824 | R²=0.9638 | R²=0.9758 |
| Gradient | | b=12.617 | b=13.1 | b=11.65 |
| a/b | | 0.73 | 0.26 | 0.49 |

It was found out from test results as to the annular orally disintegrating tablets in Examples 6 to 8 and the disk-shaped orally disintegrating tablets in Comparative Examples 6 to 8 shown in Table 8 and FIGS. 14 to 16 that when as the disintegrating agent, a PVP (polyvinyl pyrrolidone)-based disintegrating agent such as the crospovidone and a starch-based disintegrating agent such as the corn starch and Primojel were used, in most cases, a disintegrating time of each of the annular orally disintegrating tablets became shorter than a disintegrating time of each of the disk-shaped orally disintegrating tablets, which were formed at the same respective tableting pressures. In addition, it was found out that because when as to the relationship between each of the tableting pressures and each of the disintegrating times, a comparison (a/b) between the gradient a of a regression formula of each of the annular orally disintegrating tablets in Examples 6 to 8 and the gradient b of a regression formula of each of the disk-shaped orally disintegrating tablets in Comparative Examples 6 to 8, obtained by a single regression analysis, was made, the a/b was 0.90 or less, the disintegrating time per unit tableting pressure of each of the annular orally disintegrating tablets became shorter than the disintegrating time of each of the disk-shaped orally disintegrating tablets, and each of the annular orally disintegrating tablets had excellent disintegrating properties. It is to be noted that although as a tableting pressure which allows excellent disintegrating properties to be exhibited while a form as the tablet is maintained, 5 kN or more is sufficient, in consideration of a hardness and the like of each of the actually formed tablets, it is more preferable that the tableting pressure is 5 kN to 14 kN.

### 4. Influence exerted by a dimension of an external diameter of each annular orally disintegrating tablet

### (1) Manufacturing of orally disintegrating tablets

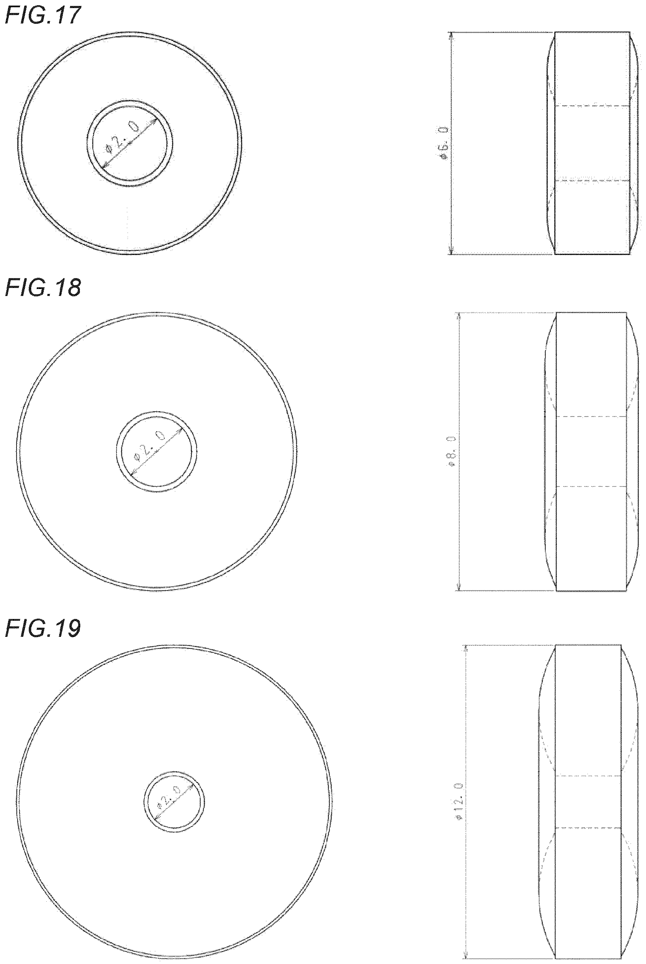

In order to study influence exerted on disintegrating times of annular orally disintegrating tablets each having a hole having the same internal diameter and disk-shaped orally disintegrating tablets, each of which was formed at the same tableting pressure, by a dimension of an external diameter of each of the orally disintegrating tablets, the below-described test was conducted. Specifically, annular orally disintegrating tablets in Examples 11 (FIG. 17), 12 (FIG. 18), and 13 (FIG. 19), each of which had an internal diameter of 2 mm and a tablet weight of 360 mg and whose external diameters were 6 mm, 8 mm, and 12 mm, were manufactured by employing the above-described same manufacturing method in which the annular orally disintegrating tablet in Example 4 was manufactured and under the same manufacturing conditions as those under which the annular orally disintegrating tablet in Example 4 was manufactured, except that the external diameters of the annular orally disintegrating tablets were 6 mm, 8 mm, and 12 mm. In addition, the disk-shaped orally disintegrating tablets in Comparative Examples 11, 12, and 13, each of which had a tablet weight of 360 mg and whose external diameters were 6 mm, 8 mm, and 12 mm, were manufactured by employing the above-described same manufacturing method in which the disk-shaped orally disintegrating tablet in Comparative Example 4 was manufactured under the same manufacturing conditions as those under which the disk-shaped orally disintegrating tablet in Comparative Example 4 was manufactured, except that the external diameters of the disk-shaped orally disintegrating tablets were 6 mm, 8 mm, and 12 mm.

Components of the annular orally disintegrating tablets (see FIGS. 17, 18, 2, and 19) in Examples 11, 12 ,4, and 13, whose external diameters were 6 mm, 8 mm, 10 mm, and 12 mm, and components of the disk-shaped orally disintegrating tablets in Comparative Examples 11, 12 ,4, and 13 are shown in Table 9.

**[Table 9]**

| (Blended amount: mg) | | | | | | | | | |
|---|---|---|---|---|---|---|---|---|---|
| | | Example 11 | Example 12 | Example 4 | Example 13 | Comparative Example 11 | Comparative Example 12 | Comparative Example 4 | Comparative Example 13 |
| Shape (external diameter/internal diameter: mm) | | Annular/with a hole (6/2) | Annular/with a hole (8/2) | Annular/with a hole (10/2) | Annular/with a hole (12/2) | Disk-shaped/without a hole (6/-) | Disk-shaped/without a hole (8/-) | Disk-shaped/without a hole (10/-) | Disk-shaped/without a hole (12/-) |
| Intra-granular part | Olmesartan medoxomil | 10 | 20 | 40 | 80 | 10 | 20 | 40 | 80 |
| | Lactose hydrate | 10 | 20 | 40 | 80 | 10 | 20 | 40 | 80 |
| | Hydroxypropyl cellulose | 0.25 | 0.5 | 1 | 2 | 0.25 | 0.5 | 1 | 2 |
| Intra-granular part total | | 20.25 | 40.5 | 81 | 162 | 20.25 | 40.5 | 81 | 162 |
| Extra-granular part | Mannitol | 59.85 | 119.7 | 239.4 | 478.8 | 59.85 | 119.7 | 239.4 | 478.8 |
| | Crospovidone (10 wt%) | 9 | 18 | 36 | 72 | 9 | 18 | 36 | 72 |
| | Magnesium stearate | 0.9 | 1.8 | 3.6 | 7.2 | 0.9 | 1.8 | 3.6 | 7.2 |
| Total (tablet weight) | | 90 | 180 | 360 | 720 | 90 | 180 | 360 | 720 |

### (2) Disintegrating properties of orally disintegrating tablets

As to the disintegrating properties of the annular orally disintegrating tablets in Examples 11, 12 ,4, and 13 and the disk-shaped orally disintegrating tablets in Comparative Examples 11, 12 ,4, and 13, as described in the above-mentioned "1. Effect of making a shape of an orally disintegrating tablet annular (with a hole)", one evaluation was conducted by comparing disintegrating times in accordance with the Disintegration Test method of the Japanese Pharmacopeia 16th Edition. As to each of the annular orally disintegrating tablets and each of the disk-shaped orally disintegrating tablets, the other evaluation was conducted by obtaining a ratio (a/b) of the disintegrating time per unit tableting pressure obtained from the relationship between the tableting pressure and the disintegrating time.

It is to be noted that tableting pressures used to study the disintegrating properties of the disintegrating tablets were 4 kN, 6 kN, and 9 kN at which the disintegrating tablets in Example 11 and Comparative Example 11 were formed; were 4 kN, 7 kN, 10 kN, and 13 kN at which the disintegrating tablet in Example 12 and Comparative Example 12 were formed; were 5 kN, 8 kN, 11 kN, and 14 kN at which the disintegrating tablets in Example 4 and Comparative Example 4 were formed; and were 7 kN, 10 kN, 13 kN, and 16 kN at which the disintegrating tablets in Example 13 and Comparative Example 13 were formed.

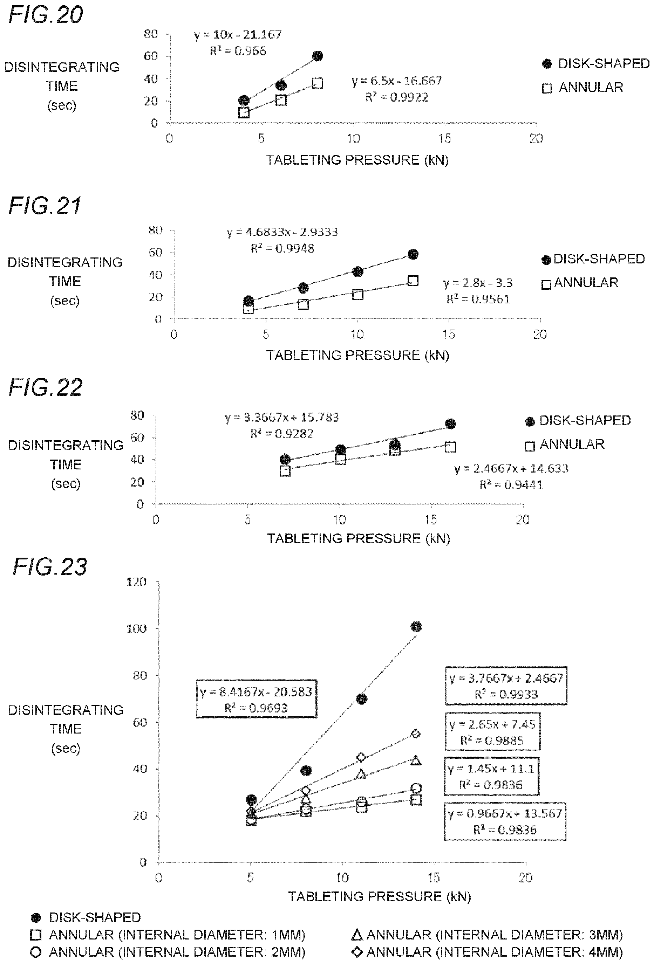

Evaluation results of the disintegrating properties as to the annular orally disintegrating tablets in Examples 11, 12, 4, and 13 and the disk-shaped orally disintegrating tablet in Comparative Examples 11, 12 ,4, and 13 are shown in Table 10 and FIGS. 20, 21, 13, and 22.

**[Table 10]**

| | Example 11 | Example 12 | Example 4 | Example 13 |
|---|---|---|---|---|
| Shape (external diameter/internal diameter: mm) | Annular/with a hole (6/2) | Annular/with a hole (8/2) | Annular/with a hole (10/2) | Annular/with a hole (12/2) |
| Disintegrating time (seconds) *(tableting pressure) | 10 (4 kN) | 10 (4 kN) | 19 (5 kN) | 30 (7 kN) |
| | 21 (6 kN) | 14 (7 kN) | 23 (8 kN) | 41 (10 kN) |
| | 36 (8 kN) | 23 (10 kN) | 26 (11 kN) | 49 (13 kN) |
| | - | 35 (13 kN) | 32 (14 kN) | 52 (16 kN) |
| Regression formula (correlation coefficient R²) | y=6.5x-16.667 | y=2.8x-3.3 | y=1.45x+11.1 | y=2.4667x+14.633 |
| | R²=0.9922 | R²=0.9561 | R²=0.9836 | R²=0.9441 |

| Gradient | a=6.5 | a=2.8 | a=1.45 | a=2.4667 |
|---|---|---|---|---|
| | Comparative Example 11 | Comparative Example 12 | Comparative Example 4 | Comparative Example 13 |
| Shape (external diameter/internal diameter: mm) | Disk-shaped /without a hole (6/-) | Disk-shaped /without a hole (8/-) | Disk-shaped /without a hole (10/-) | Disk-shaped /without a hole (12/-) |
| Disintegrating time (seconds) *(tableting pressure) | 21 (4 kN) | 17 (4 kN) | 27 (5 kN) | 41 (7 kN) |
| | 35 (6 kN) | 29 (7 kN) | 40 (8 kN) | 50 (10 kN) |
| | 61 (8 kN) | 43 (10 kN) | 70 (11 kN) | 55 (13 kN) |
| | - | 59 (13 kN) | 101 (14 kN) | 73 (16 kN) |
| Regression formula (correlation coefficient R²) | y=10x-21.167 | y=4.6833x-2.9333 | y=8.4167x-20.583 | y=3.3667x+15.783 |
| | R²=0.966 | R²=0.9948 | R²=0.9693 | R²=0.9282 |
| Gradient | b=10 | b=4.6833 | b=8.4167 | b=3.3667 |
| a/b | 0.65 | 0.60 | 0.17 | 0.73 |

It was found out from Table 10 and FIGS. 20, 21, 13, and 22 that in a case where the internal diameter of each of the annular orally disintegrating tablets was made constant and to be 2 mm, when the external diameter was within a range of 6 mm to 12 mm, the disintegrating time of each of the annular orally disintegrating tablets became shorter than the disintegrating time of each of the disk-shaped orally disintegrating tablets compression-formed at the same respective tableting pressures and had the same respective weights and external diameters. In addition, it was found out that even when each of tableting pressures was increased, each of the annular orally disintegrating tablets in Examples 11, 12 ,4, and 13 allowed a delay of disintegration to be more suppressed than each of the disk-shaped orally disintegrating tablets in Comparative Examples 11, 12 ,4, and 13. It is to be noted that although as a tableting pressure which allows excellent disintegrating properties to be exhibited while a form as the tablet is maintained, 4 kN or more is sufficient, in consideration of a hardness and the like of each of the actually formed tablets, it is more preferable that the tableting pressure is 4 kN to 16 kN.

Furthermore, it was found out that because when as to the relationship between each of the tableting pressures and each of the disintegrating times, a comparison (a/b) between the gradient a of a regression formula of each of the annular orally disintegrating tablets in Examples 11, 12 ,4, and 13 and the gradient b of a regression formula of each of the disk-shaped orally disintegrating tablets in Comparative Examples 11, 12 ,4, and 13, obtained by a single regression analysis, was made, the a/b was 0.80 or less, the disintegrating time per unit tableting pressure of each of the annular orally disintegrating tablets became shorter than the disintegrating time of each of the disk-shaped orally disintegrating tablets, and in particular, in a case of the annular orally disintegrating tablet which had the internal diameter of 2 mm and the external diameter of 10 mm, a ratio (a/b) of the gradient was 0.17, the above-mentioned annular orally disintegrating tablet had extremely excellent disintegrating properties.

### 5. Influence exerted by a dimension of an internal diameter of each annular orally disintegrating tablet

### (1) Manufacturing of orally disintegrating tablets

In order to study influence exerted on disintegrating times of each of annular orally disintegrating tablets and a disk-shaped orally disintegrating tablet, which were formed at the same respective tableting pressures, by a dimension of an internal diameter of a hole of each of the annular orally disintegrating tablets, the below-described test was conducted. Specifically, annular orally disintegrating tablets in Examples 14, 15, and 16, whose each external diameter was 10 mm, whose respective internal diameters were 1 mm (FIG. 1), 3 mm (FIG. 3), and 4 mm (FIG. 4), and whose each tablet weight was 360 mg, were manufactured by employing the above-described same manufacturing method in which the annular orally disintegrating tablet in Example 4 was manufactured and under the same manufacturing conditions as those under which the annular orally disintegrating tablet in Example 4 was manufactured, except that the respective internal diameters of holes of the annular orally disintegrating tablets were made to be 1 mm, 3 mm, and 4 mm.

Components of the annular orally disintegrating tablets in Examples 14, 4, 15, and 16 (see FIGS. 1 to 4) whose internal diameters of the holes were made to be 1 mm, 2 mm, 3 mm, and 4 mm and components of the disk-shaped orally disintegrating tablet in Comparative Example 4 (see FIG. 5) are shown in Table 11.

**[Table 11]**

| (Blended amount: mg) | | | | | | |
|---|---|---|---|---|---|---|
| | | Example 14 | Example 4 | Example 15 | Example 16 | Comparative Example 4 |
| Shape (external diameter/internal diameter: mm) | | Annular /with a hole (10/1) | Annular /with a hole (10/2) | Annular /with a hole (10/3) | Annular /with a hole (10/4) | Disk-shaped /without a hole (10/-) |
| Intra-granular part | Olmesartan medoxomil | 40 | | | | |
| | Lactose hydrate | 40 | | | | |
| | Hydroxypropyl cellulose | 1 | | | | |
| Intra-granular part total | | 81 | | | | |
| Extra-granular part | Mannitol | 239.4 | | | | |
| | Crospovidone (10 wt%) | 36 | | | | |
| | Magnesium stearate | 3.6 | | | | |
| Total (tablet weight) | | 360 | | | | |

### (2) Disintegrating properties of orally disintegrating tablets

As to disintegrating properties of the annular orally disintegrating tablets in Examples 14, 4, 15, and 16 and the disk-shaped orally disintegrating tablet in Comparative Example 4, as described in the above-mentioned "1. Effect of making a shape of an orally disintegrating tablet annular (with a hole)", one evaluation was conducted by comparing disintegrating times in accordance with the Disintegration Test method of the Japanese Pharmacopeia 16th Edition, and the other evaluation was conducted by comparing the gradient a of each of the annular orally disintegrating tablets in Examples 14, 4, 15, and 16, which was a disintegrating time per unit tableting pressure, obtained from the relationship between a tableting pressure upon compression forming and a disintegrating time and the gradient b of the disk-shaped orally disintegrating tablet in Comparative Example 4.

Evaluation results of disintegrating properties of the annular orally disintegrating tablets in Examples 14, 4, 15, and 16 and the disk-shaped orally disintegrating tablet in Comparative Example 4 are shown in Table 12 and FIG. 23.

**[Table 12]**

| | | Example 14 | Example 4 | Example 15 | Example 16 | Comparative Example 4 |
|---|---|---|---|---|---|---|
| Shape (external diameter/internal diameter: mm) | | Annular/with a hole (10/1) | Annular/with a hole (10/2) | Annular/with a hole (10/3) | Annular/with a hole (10/4) | Disk-shaped/without a hole (10/-) |
| Disintegrating time (seconds) | Tableting pressure 5 kN | 19 | 19 | 21 | 22 | 27 |
| | Tableting pressure 8 kN | 23 | 23 | 28 | 31 | 40 |
| | Tableting pressure 11 kN | 26 | 26 | 38 | 45 | 70 |
| | Tableting pressure 14 kN | 32 | 32 | 44 | 55 | 101 |
| Regression formula (correlation coefficient R²) | | y=1.45x+11.1 | y=1.45x+11.1 | y=2.65x+7.45 | y=3.7667x+2.4667 | y=8.4167x+20.583 |
| | | R²=0.9836 | R²=0.9836 | R²=0.9885 | R²=0.9933 | R²=0.9693 |
| Gradient | | a=1.45 | a=1.45 | a=2.65 | a=3.7667 | b=8.4167 |

It was found out from Table 12 and FIG. 23 that the smaller the internal diameter of the hole of each of the annular orally disintegrating tablets is, the shorter the disintegrating time of each of the annular orally disintegrating tablets formed at the same respective tableting pressures is. In addition, also as to the disintegrating time per unit tableting pressure obtained from the relationship between the tableting pressure and the disintegrating time, although the gradient a of each of the annular orally disintegrating tablets in Examples 14, 4, 15, and 16 became small in accordance with a decrease in the internal diameter of the hole, the gradient b of the disk-shaped orally disintegrating tablet in Comparative Example 4 which had no hole did not become smaller than the gradient a of each of the annular orally disintegrating tablets in Examples 14, 4, 15, and 16, which had the hole, and the disk-shaped orally disintegrating tablet was not capable of shortening the disintegrating time, unlike the annular orally disintegrating tablets. It is to be noted that although as a tableting pressure which allows excellent disintegrating properties to be exhibited while a form as the tablet is maintained, 5 kN or more is sufficient, in consideration of a hardness and the like of each of the actually formed tablets, it is more preferable that the tableting pressure is 5 kN to 14 kN.

In addition, it was found from Tables 10 and 12 that in order to cause the excellent disintegrating properties as described above to be exhibited, by making a ratio of the external diameter and the internal diameter of the annular orally disintegrating tablet of the present invention to be 10 : 1 to 6 : 2 or more preferably, making the ratio thereof to be 10 : 1 to 10 : 4 and making the internal diameter of the annular orally disintegrating tablet to be larger than 0 mm and 4 mm or less, it was made possible to suppress the disintegrating time within 60 seconds.

### 6. Influence exerted by providing an orally disintegrating tablet with a score line

### (1) Manufacturing of orally disintegrating tablets

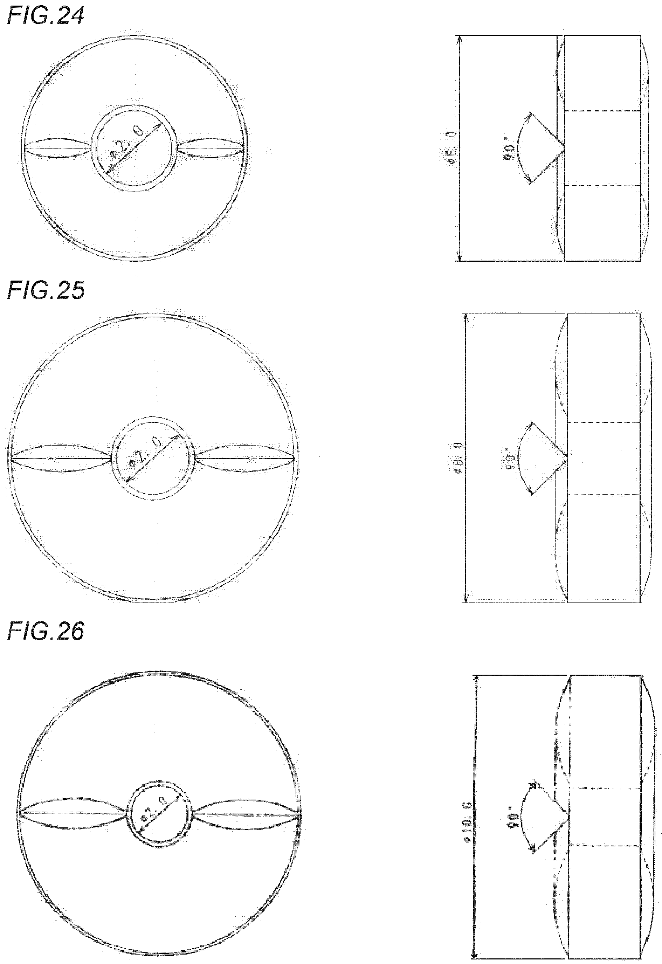

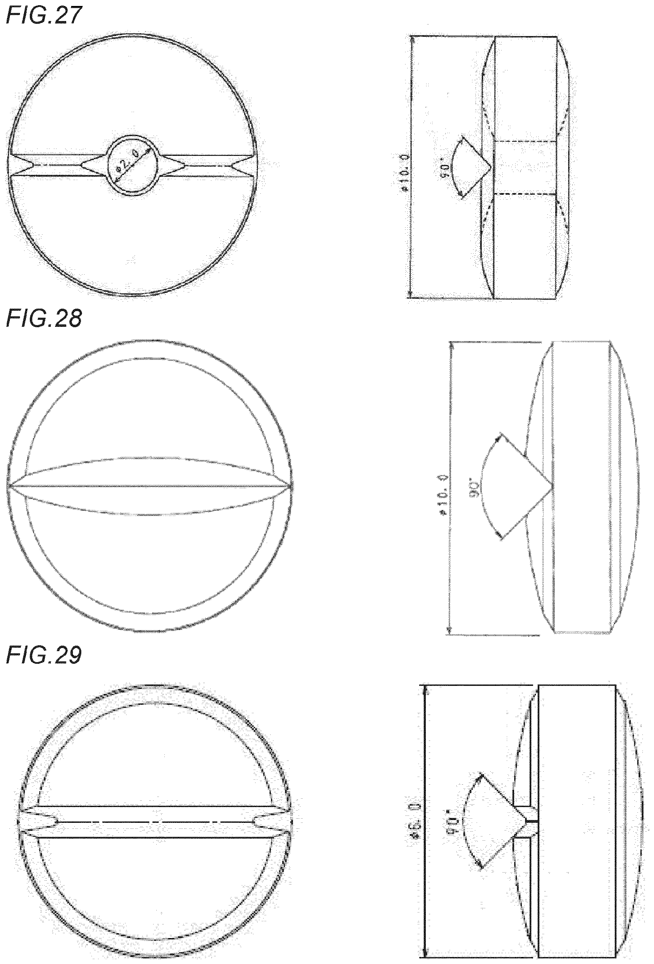

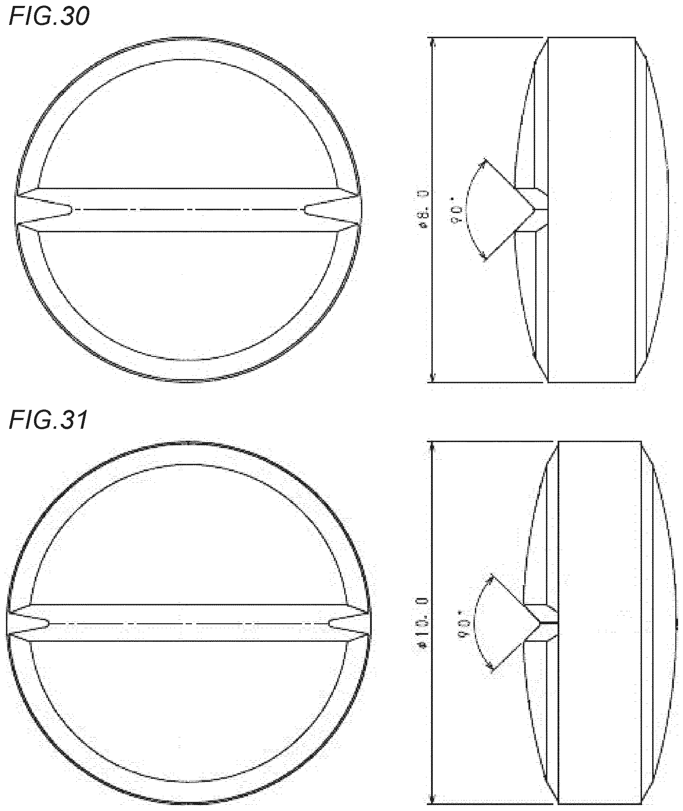

Annular orally disintegrating tablets (FIG. 26) in Examples 19R and 22R, in each of which as a drug, olmesartan medoxomil or azilsartan was used and each of which had a round score line and annular orally disintegrating tablets (FIG. 27) in Examples 19S and 22S, in each of which as the drug, the olmesartan medoxomil or the azilsartan was used and each of which had a straight score line; and disk-shaped orally disintegrating tablets (FIG. 28) in Comparative Examples 16R and 20R, each of which a round score line and disk-shaped orally disintegrating tablets (FIG. 31) in Comparative Examples 16S and 20S, each of which had a straight score line were manufactured as described below.

Among the above-mentioned Examples and Comparative Examples, the annular orally disintegrating tablets in Examples 19R and 19S, in each of which as the drug, the olmesartan medoxomil was used were manufactured by using the above-described same prescription (see Table 5) as that for the annular orally disintegrating tablet in Example 4 and by employing the above-described same manufacturing method in which the annular orally disintegrating tablet in Example 4 was manufactured, except that each of the annular orally disintegrating tablets in Examples 19R and 19S had the round score line or the straight score line. In addition, the disk-shaped orally disintegrating tablets in Comparative Examples 16R and 16S in which as the drug, the olmesartan medoxomil was used were manufactured by using the above-described same prescription (see Table 5) as that for the disk-shaped orally disintegrating tablet in Comparative Example 4 and by employing the above-described same manufacturing method in which the disk-shaped orally disintegrating tablet in Comparative Example 4 was manufactured, except that each of the disk-shaped orally disintegrating tablets had no hole.

In addition, among the above-mentioned Examples and Comparative Examples, the annular orally disintegrating tablets in Examples 22R and 22S, in each of which as the drug, the azilsartan was used were manufactured by using the above-described same prescription (see Table 2) as that for the annular orally disintegrating tablet in Example 9 and by employing the above-described same manufacturing method in which the annular orally disintegrating tablet in Example 9 was manufactured, except that each of the annular orally disintegrating tablets had the round score line or the straight score line. In addition, the disk-shaped orally disintegrating tablets in Comparative Examples 20R and 20S, in each of which as the drug, the azilsartan was used were manufactured by using the above-described same prescription (see Table 2) as that for the disk-shaped orally disintegrating tablet in Comparative Example 9 and by employing the above-described same manufacturing method in which the disk-shaped orally disintegrating tablet in Comparative Example 9 was manufactured, except that each of the disk-shaped orally disintegrating tablets in Comparative Examples 20R and 20S had the round score line or the straight score line.

### (2) Scoring properties of orally disintegrating tablets

As to 10 tablets of each of orally disintegrating tablets, scoring properties of orally disintegrating tablets were evaluated by standard deviation (%) and acceptance values (%) calculated in accordance with "2. Mass Variation Test" in "6.02 Uniformity of Dosage Units" described in the Japanese Pharmacopeia 17th Edition. The smaller the standard deviation (%) is, the smaller variation of mass of each tablet piece after scoring is, and the smaller the acceptance value (%) is, the more readily the scoring can be made, meaning that the scoring properties are excellent.

As described below, the scoring properties of each of the annular orally disintegrating tablets, in each of which the olmesartan medoxomil or the azilsartan was used and each of which had the score line, and the scoring properties of each of the disk-shaped orally disintegrating tablets, in each of which the olmesartan medoxomil or the azilsartan was used and each of which had the score line, were evaluated by comparing the standard deviation (%) and the acceptance values (%) calculated in accordance with the Mass Variation Test in the Uniformity of Dosage Units described in the Japanese Pharmacopeia 17th Edition.

Evaluation results of the scoring properties as to the annular orally disintegrating tablets in Examples 19R and 22R, each of which had the round score line, the disk-shaped orally disintegrating tablets in Comparative Examples 16R and 20R, each of which had the round score line, the annular orally disintegrating tablets in Examples 19S and 22S, each of which had the straight score line, and the disk-shaped orally disintegrating tablets in Comparative Examples 16S and 20S, each of which had the straight score line are shown in Table 13 and FIG. 34.

**[Table 13]**

| | | Example 19R | Comparative Example 16R | Example 22R | Comparative Example 20R | Example 19S | Comparative Example 16S | Example 22S | Comparative Example 20S |
|---|---|---|---|---|---|---|---|---|---|
| Drug | | Olmesartan medoxomil | | Azilsartan | | Olmesartan medoxomil | | Azilsartan | |
| Shape | Score line shape | Round score line | | | | Straight score line | | | |
| | Tablet shape | Annular | Disk-shaped | Annular | Disk-shaped | Annular | Disk-shaped | Annular | Disk-shaped |
| | External diameter (mm) | 10 | 10 | 10 | 10 | 10 | 10 | 10 | 10 |
| | Internal diameter (mm) | 2 | - | 2 | - | 2 | - | 2 | - |
| | Tablet weight (mg) | 360 | | | | 360 | | | |
| Tableting pressure (kN) | | 8 | | 6 | | 8 | | 6 | |
| Evaluation | Range of mass of scored tablet (%) | 96.9 to 102.7 | 91.0 to 105.8 | 89.9 to 103.0 | 73.1 to 126.9 | 98.1 to 103.0 | 90.4 to 107.7 | 92.3 to 107.7 | 86.7 to 139.0 |
| | Standard deviation (%) | 2.2 | 5.9 | 5.4 | 17.4 | 1.8 | 5.8 | 6.1 | 15.3 |
| | Acceptance value (%) | **5.3** | **14.2** | **12.9** | **41.8** | **4.3** | **13.9** | **14.7** | **36.7** |

It was found out from Table 13 and FIG. 34 that regardless of whether the drug of the tablet was the olmesartan medoxomil or the azilsartan, the standard deviation (%) and the acceptance values (%) of the annular orally disintegrating tablets in Examples 19R, 22R, 19S, and 22S, each of which had the round score line or the straight score line, became smaller than the standard deviation (%) and the acceptance values (%) of the disk-shaped orally disintegrating tablets in Comparative Examples 16R, 20R, 16S, and 20S, each of which had the round score line or the straight score line and which were formed under the same conditions under which the annular orally disintegrating tablets in Examples 19R, 22R, 19S, and 22S were formed.

In addition, it was found out that in a case where the same drug was used, regardless of whether the score line was the round score line or the straight score line, the standard deviation (%) and the acceptance values (%) of the annular orally disintegrating tablets in Examples 19R and 19S, which were formed under the same conditions, were substantially the same as each other; the standard deviation (%) and the acceptance values (%) of the annular orally disintegrating tablets in Examples 22R and 22S, which were formed under the same conditions, were substantially the same as each other; the standard deviation (%) and the acceptance values (%) of the disk-shaped orally disintegrating tablets in Comparative Examples 16R and 16S, which were formed under the same conditions, were substantially the same as each other; and the standard deviation (%) and the acceptance values (%) of the disk-shaped orally disintegrating tablets in Comparative Examples 20R and 20S , which were formed under the same conditions, were substantially the same as each other.

In other words, it was found out that although regardless of the kinds of the drugs and the kinds of the score lines, each of the annular orally disintegrating tablets, which had the score line, had the same strength, which allowed a form as the tablet, as the strength of each of the disk-shaped orally disintegrating tablets, which were formed at the same respective tableting pressures and had the score lines, the annular orally disintegrating tablets exhibited more excellent scoring properties than those of the disk-shaped orally disintegrating tablets. It is to be noted that each of the score lines used in the present invention is a V-shaped groove whose apex angle θ is within a range of 90° ± 20° in a cross-sectional view.

### 7. Influence exerted by a dimension of an external diameter of each annular orally disintegrating tablet having a score line

### (1) Manufacturing of orally disintegrating tablets

In order to study influence exerted on scoring properties of annular orally disintegrating tablets, each of which had a hole having the same internal diameter and the same score line, and disk-shaped orally disintegrating tablets, each of which had the same score line, by an external diameter of each of the tablets, the below-described test was conducted. Specifically, annular orally disintegrating tablets in Examples 17R (FIG. 24) and 18R (FIG. 25), each of which has a round score line, were manufactured by using the above-described same prescription as that for the annular orally disintegrating tablet (the external diameter of 10 mm and the weight of 360 mg) in Example 19R and by employing the above-described same manufacturing method in which the annular orally disintegrating tablet in Example 19R was manufactured, except that external diameters thereof were 6 mm (a weight of 90 mg) and 8 mm (a weight of 180 mg). In addition, disk-shaped orally disintegrating tablets in Comparative Examples 14S (FIG. 29), 15S (FIG. 30), and 16S (FIG. 31) were manufactured by using the above-described the same prescription as that for each of the above-mentioned annular orally disintegrating tablets in Examples 17R, 18R, and 19R and by employing the above-described same manufacturing method in which each of the annular orally disintegrating tablets in Examples 17R, 18R, and 19R was manufactured, except that each of the disk-shaped orally disintegrating tablets had no hole in a central portion thereof and had a straight score line.

### (2) Scoring properties of orally disintegrating tablets

As to scoring properties of the annular orally disintegrating tablets in Examples 17R, 18R, and 19R and the disk-shaped orally disintegrating tablets in Comparative Examples 14S, 15S, and 16S, evaluation was conducted by the same test method and calculation method as those described in the above-mentioned "6. (2) Scoring properties of orally disintegrating tablets" based on the Japanese Pharmacopeia 17th Edition.

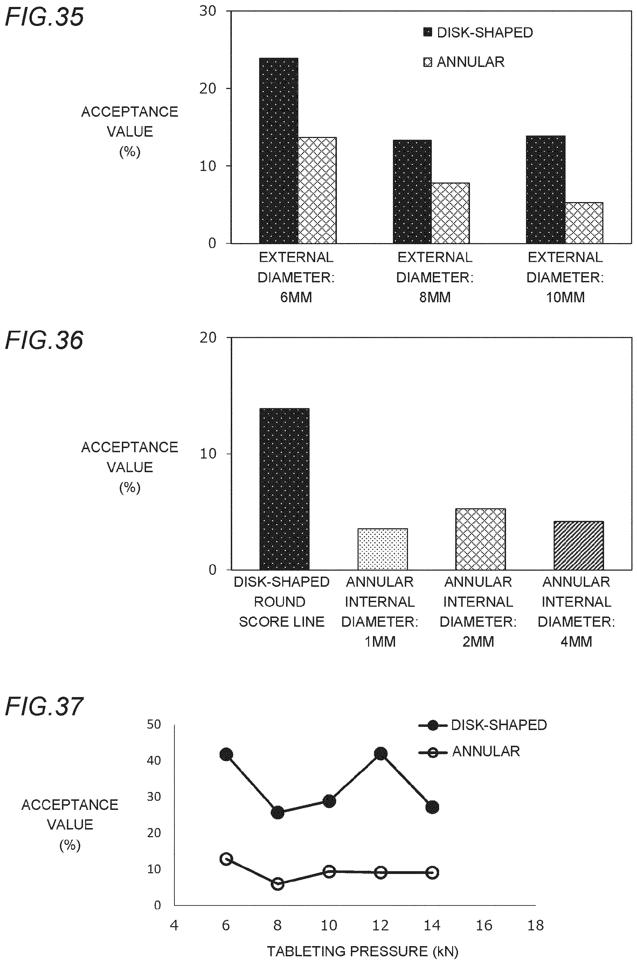

Evaluation results as to the scoring properties of the annular orally disintegrating tablets in Examples 17R, 18R, and 19R, each of which had the round score line and the scoring properties of the disk-shaped orally disintegrating tablets in Comparative Examples 14S, 15S, and 16S, each of which had the straight score line are shown in Table 14 and FIG. 35.

**[Table 14] 48**

| | | Example 17R | Example 18R | Example 19R |
|---|---|---|---|---|
| Drug | | Olmesartan medoxomil | | |
| Shape | Score line shape | Round score line | | |
| | Tablet shape | Annular | | |
| | External diameter (mm) | **6** | **8** | **10** |
| | Internal diameter (mm) | 2 | | |
| | Tablet weight (mg) | 90 | 180 | 360 |
| Tableting pressure (kN) | | 4 | 6 | 8 |
| Evaluation | Range of mass of scored tablet (%) | 92.9 to 109.1 | 98.1 to 105.8 | 96.9 to 102.7 |
| | Standard deviation | 5.7 | 3.2 | 2.2 |
| | Acceptance value (%) | **13.7** | **7.8** | **5.3** |

| | | Comparative Example 14S | Comparative Example 15S | Comparative Example 16S |
|---|---|---|---|---|
| Drug | | Olmesartan medoxomil | | |
| Shape | Score line shape | Straight score line | | |
| | Tablet shape | Disk-shaped | | |
| | External diameter (mm) | **6** | **8** | **10** |
| | Internal diameter (mm) | | | |
| | Tablet weight (mg) | 90 | 180 | 360 |
| Tableting pressure (kN) | | 4 | 6 | 8 |
| Evaluation | Range of mass of scored tablet (%) | 86.0 to 115.6 | 92.7 to 108.3 | 90.4 to 107.7 |
| | Standard deviation | 9.9 | 5.5 | 5.8 |
| | Acceptance value (%) | **23.9** | **13.3** | **13.9** |

It was found out from Table 14 and FIG. 35 that as long as the internal diameter of each of the annular orally disintegrating tablets, which had the round score line, was constant (for example, 2 mm), regardless of the external diameters (for example, within a range of 6 mm to 10 mm), as to any of the external diameters, the annular orally disintegrating tablets exhibited more excellent scoring properties than those of the disk-shaped orally disintegrating tablets were compression-formed at the same respective tableting pressures and had the same weight and the straight score line.

In addition, in the case where the drug was the olmesartan medoxomil, the comparison between the annular orally disintegrating tablets in Examples 17R, 18R, and 19R, each of which had the round score line, and the disk-shaped orally disintegrating tablets in Comparative Examples 14S, 15S, and 16S, each of which had the straight score line, was made. In consideration of the results obtained in the above-mentioned "6. (2) Scoring properties of orally disintegrating tablets", it is considered that as long as the internal diameter is constant (for example, 2 mm), regardless of the kinds of the drugs, the kinds of the score lines, and the external diameters (for example, within a range of 6 mm to 10 mm), as to any of the external diameter, the annular orally disintegrating tablets, each of which has the score line, exhibit more excellent scoring properties than those of the disk-shaped orally disintegrating tablets, each of which has the same score line.

### 8. Influence exerted by a dimension of an internal diameter of each annular orally disintegrating tablet having a score line

### (1) Manufacturing of orally disintegrating tablets

In order to study influence exerted on scoring properties of annular orally disintegrating tablets, each of which had the same external diameter and score line and was formed at the same tableting pressure, and scoring properties of a disk-shaped orally disintegrating tablets by a dimension of an internal diameter of a hole of each of the annular orally disintegrating tablets, the below-described test was conducted. Specifically, annular orally disintegrating tablets in Example 20R (FIG. 32) and 21R (FIG. 33), each of which had a round score line, were manufactured by using the above-described same prescription as that for the annular orally disintegrating tablet (an internal diameter of 2 mm) in Example 19R and by employing the above-described same manufacturing method in which the annular orally disintegrating tablet in Example 19R was manufactured, except that internal diameters of holes were made to be 1 mm and 4 mm. In addition, the disk-shaped orally disintegrating tablet in Comparative Example 16S was manufactured as described in the above-mentioned "7. (1) Manufacturing of orally disintegrating tablets".

### (2) Scoring properties of orally disintegrating tablets

As to the scoring properties of the annular orally disintegrating tablets in Examples 20R, 19R, and 21R, each of which had the round score line and the scoring properties of the disk-shaped orally disintegrating tablet in Comparative Example 16S, evaluation was conducted by the same test method and calculation method as those described in the above-mentioned "6. (2) Scoring properties of orally disintegrating tablets" based on the Japanese Pharmacopeia 17th Edition.

Evaluation results as to the scoring properties of the annular orally disintegrating tablets in Examples 20R, 19R, and 21R, each of which has the round score line and the scoring properties of the disk-shaped orally disintegrating tablet in Comparative Example 16S which had the straight score line are shown in Table 15 and FIG. 36.

**[Table 15]**

| | | Comparative Example 16S | Example 20R | Example 19R | Example 21R |
|---|---|---|---|---|---|
| | Drug | Olmesartan medoxomil | | | |
| Shape | Score line shape | Straight score line | Round score line | | |
| | Tablet shape | Disk-shaped | Annular | | |
| | External diameter (mm) | 10 | | | |
| | Internal diameter (mm) | - | **1** | **2** | **4** |
| | Tablet weight (mg) | 360 | | | |
| Tableting pressure (kN) | | 8 | | | |
| Evaluation | Range of mass of scored tablet (%) | 90.4 to 107.7 | 97.7 to 103.0 | 96.9 to 102.7 | 98.1 to 104.6 |
| | Standard deviation (%) | 5.8 | 1.5 | 2.2 | 1.7 |
| | Acceptance value (%) | **13.9** | **3.5** | **5.3** | **4.2** |

It was found out from Table 15 and FIG. 36 that regardless of the internal diameters (for example, within a range of 1 mm to 4 mm), the annular orally disintegrating tablets, each of which had the round score line exhibited more excellent scoring properties than those of the disk-shaped orally disintegrating tablet which was compression-formed at the same tableting pressure and had the same weight and external diameter and the straight score line.

In addition, as to a case where the drug was the olmesartan medoxomil, a comparison between the annular orally disintegrating tablets in Examples 20R, 19R, and 21R, each of which has the round score line, and the disk-shaped orally disintegrating tablet in Comparative Example 16S which had the straight score line was made. In consideration of the results obtained in the above-mentioned "6. Influence exerted by providing an orally disintegrating tablet with a score line", it is considered that regardless of the kind of the drug, the kinds of the score lines, and the internal diameters (for example, within a range of 1 mm to 4 mm), the annular orally disintegrating tablets, each of which has the score line, exhibit more excellent scoring properties than the scoring properties of the disk-shaped orally disintegrating tablet which has the same score line.

### 9. Influence exerted by a tableting pressure at which each annular orally disintegrating tablet having a score line is formed

### (1) Manufacturing of orally disintegrating tablets

In order to study influence exerted on scoring properties of annular orally disintegrating tablets and disk-shaped orally disintegrating tablets, each of which was formed under the same conditions and had the same score line, by a dimension of a tableting pressure at which each of the orally disintegrating tablets was formed, the below-described test was conducted. Specifically, annular orally disintegrating tablets (FIG. 26) in Examples 23R, 24R, 25R, and 26R were manufactured by using the above-described same prescription as that for the annular orally disintegrating tablet (the tableting pressure 6 kN) in Example 22R and by employing the above-described same manufacturing method in which the annular orally disintegrating tablet in Example 22R was manufactured, except that tableting pressures were 8 kN, 10 kN, 12 kN, and 14 kN.

In addition, disk-shaped orally disintegrating tablets (FIG. 28) in Comparative Examples 21R, 22R, 23R, and 24R, each of which had a round score line were manufactured by using the above-described same prescription as that for the annular orally disintegrating tablet (the tableting pressure 6 kN) in Comparative Example 20R and by employing the above-described same manufacturing method in which the annular orally disintegrating tablet in Comparative Example 20R was manufactured, except that tableting pressures were 8 kN, 10 kN, 12 kN, and 14 kN.

### (2) Scoring properties of orally disintegrating tablets

As to the scoring properties of the annular orally disintegrating tablets in Examples 22R, 23R, 24R, 25R, and 26R, each of which had the round score line and those of the disk-shaped orally disintegrating tablets in Comparative Examples 20R, 21R, 22R, 23R, and 24R, each of which had the round score line, evaluation was conducted by the same test method and calculation method as those described in the above-mentioned "6. (2) Scoring properties of orally disintegrating tablets" based on the Japanese Pharmacopeia 17th Edition.

Evaluation results as to the scoring properties of the annular orally disintegrating tablet in Examples 22R, 23R, 24R, 25R, and 26R, each of which had the round score line and those of the disk-shaped orally disintegrating tablets in Comparative Examples 20R, 21R, 22R, 23R, and 24R, each of which had the round score line, are shown in Table 16 and FIG. 37.

**[Table 16]**

| | | Example 22R | Example 23R | Example 24R | Example 25R | Example 26R |
|---|---|---|---|---|---|---|
| | Drug | Azilsartan | | | | |
| Shape | Score line shape | Round score line | | | | |
| | Tablet shape | Annular | | | | |
| | External diameter (mm) | 10 | | | | |
| | Internal diameter (mm) | 2 | | | | |
| | Tablet weight (mg) | 360 | | | | |
| Tableting pressure (kN) | | **6** | **8** | **10** | **12** | **14** |
| Evaluation | Range of mass of scored tablet (%) | 89.9 to 103.0 | 96.3 to 103.4 | 95.5 to 104.1 | 93.1 to 101.7 | 97.9 to 105.6 |
| | Standard deviation (%) | 5.4 | 2.5 | 3.9 | 3.8 | 3.8 |
| | Acceptance value (%) | **12.9** | **6.0** | **9.4** | **9.1** | **9.1** |

| | | Comparative Example 20R | Comparative Example 21R | Comparative Example 22R | Comparative Example 23R | Comparative Example 24R |
|---|---|---|---|---|---|---|
| | Drug | Azilsartan | | | | |
| Shape | Score line shape | Round score lines | | | | |
| | Tablet shape | Disk-shaped | | | | |
| | External diameter (mm) | 10 | | | | |
| | Internal diameter (mm) | - | | | | |
| | Tablet weight (mg) | 360 | | | | |
| Tableting pressure (kN) | | **6** | **8** | **10** | **12** | **14** |
| Evaluation | Range of mass of scored tablet (%) | 73.1 to 126.9 | 83.4 to 116.6 | 78.8 to 121.2 | 74.8 to 125.2 | 81.3 to 118.7 |
| | Standard deviation (%) | 17.4 | 10.7 | 12.0 | 17.5 | 11.3 |
| | Acceptance value (%) | **41.8** | **25.7** | **28.9** | **42.0** | **27.2** |

It was found out from Table 16 and FIG. 37 that regardless of the tableting pressures (for example, within a range of 6 kN to 14 kN), at any of the tableting pressures, the annular orally disintegrating tablets, each of which had the round score line, exhibited more excellent scoring properties than those of the disk-shaped orally disintegrating tablets, each of which had the same weight and external diameter and the round score line.

In addition, as to Examples 22R to 26R and Comparative Examples 20R to 24R, in the case where the drug was the olmesartan medoxomil and the score line was the round score line, the comparison between the annular orally disintegrating tablets and the disk-shaped orally disintegrating tablets was made. In consideration of the results obtained in the above-mentioned "6. Influence exerted by providing an orally disintegrating tablet with a score line", it is considered that regardless of the kinds of the drugs, the kinds of the score lines, and the tableting pressures (for example, within a range of 6 kN to 14 kN), the annular orally disintegrating tablets, each of which has the score line, exhibit more excellent scoring properties than those of the disk-shaped orally disintegrating tablets, each of which has the same score line.

It is to be noted that as described in the above-mentioned "4. Influence exerted by a dimension of an external diameter of each annular orally disintegrating tablet", in the present invention, although as a tableting pressure which is suited to maintain a form as the tablet and allows excellent scoring properties to be exhibited, 4 kN or more is sufficient, in consideration of a hardness and the like of the formed tablets, it is preferable that the tableting pressure is 4 kN to 16 kN, and in order to obtain excellent scoring properties in particular, it is more preferable that the tableting pressure is 6 kN to 14 kN.

## Claims

1. An annular orally disintegrating tablet,
containing a drug and a disintegrating agent; and
having a hole in a central portion and being annular, wherein a content of the disintegrating agent relative to a total weight of the annular orally disintegrating tablet is 2% by weight or more and 50% by weight or less.

2. The annular orally disintegrating tablet according to claim 1, wherein a disintegrating time in an oral cavity is within 60 seconds.

3. The annular orally disintegrating tablet according to claim 1 or 2, wherein a ratio between an external diameter and an internal diameter is 10 : 1 to 10 : 4.

4. The annular orally disintegrating tablet according to any one of claims 1 to 3, wherein a ratio between the external diameter and the internal diameter is 10 : 1 to 6 : 2.

5. The annular orally disintegrating tablet according to any one of claims 1 to 4, wherein the internal diameter is larger than 0 mm and 4 mm or less.

6. The annular orally disintegrating tablet according to any one of claims 1 to 5, wherein the disintegrating agent is a polyvinyl pyrrolidone-based disintegrating agent and/or a starch-based disintegrating agent.

7. The annular orally disintegrating tablet according to claim 6, wherein the polyvinyl pyrrolidone-based disintegrating agent is crospovidone.

8. The annular orally disintegrating tablet according to claim 6, wherein the starch-based disintegrating agent is corn starch or sodium starch glycolate.

9. The annular orally disintegrating tablet according to any one of claims 1 to 8, wherein the drug is olmesartan medoxomil or azilsartan.

10. The annular orally disintegrating tablet according to any one of claims 1 to 9, wherein when a gradient of the disintegrating time (seconds) of the annular orally disintegrating tablet with respect to a tableting pressure (kN) upon compression forming is defined as a and a gradient of a disintegrating time (seconds) of a disk-shaped orally disintegrating tablet with respect to a tableting pressure (kN) upon compression forming is defined as b, the disk-shaped orally disintegrating tablet having a same weight and external diameter as the weight and external diameter of the annular orally disintegrating tablet and having no hole, a ratio (a/b) between the gradient a and the gradient b is 0.90 or less.

11. The annular orally disintegrating tablet according to claim 10, wherein a ratio (a/b) between the gradient a and the gradient b is 0.50 or less.

12. The annular orally disintegrating tablet according to any one of claims 1 to 11, wherein the annular orally disintegrating tablet is compression-formed at a tableting pressure of 4 kN to 16 kN.

13. The annular orally disintegrating tablet according to any one of claims 1 to 13, on a surface of the tablet, a score line constituted of at least one groove is provided.

14. The annular orally disintegrating tablet according to claim 13, wherein the score line has a straight shape or a round shape in a plan view.

15. The annular orally disintegrating tablet according to claim 13 or 14, wherein the score line is a V-shaped groove in a cross-sectional view, an apex angle θ of the V-shaped groove being within a range of 90° ± 20°.

16. The annular orally disintegrating tablet according to any one of claims 13 to 15, wherein an external diameter is 6 mm or more and 10 mm or less.

17. The annular orally disintegrating tablet according to any one of claims 13 to 16, wherein an internal diameter is 1 mm or more and 4 mm or less.

18. The annular orally disintegrating tablet according to any one of claims 13 to 17, wherein the annular orally disintegrating tablet is compression-formed at a tableting pressure of 6 kN to 14 kN.
